# EUROPEAN PATENT APPLICATION

(11) **EP 3 967 356 A1**
(43) Date of publication of application: **16.03.2022**
(21) Application number: 19927750.0
(22) Date of filing: 04.10.2019
(51) Int. Cl.: A61M 25/09

(54) **GUIDE WIRE**

(30) Priority: 07.05.2019 WO PCT/JP2019/018256
(71) Applicant: ASAHI INTECC CO., LTD., Seto-shi, Aichi 489-0071 (JP)
(72) Inventor: UCHIMURA Sho, Seto-shi, Aichi 489-0071 (JP); CHAPOT Rene, 45219 Essen (DE)
(74) Representative: Winter, Brandl - Partnerschaft mbB
(86) International application number: PCT/JP2019/039370
(87) International publication number: WO 2020/225935

(57) **Abstract**

A guide wire includes: a core shaft; an engagement part provided in outer circumference of the core shaft; a cylindrical body provided in an outside of the core shaft and movable along an axis line direction of the core shaft; and an engaged part that is provided in the cylindrical body, and engages with the engagement part when the cylindrical body moves in a first direction along the axis line direction of the core shaft to regulate further movement of the cylindrical body in the first direction.

## Description

### Technical Field

The present invention relates to a guide wire.

### Background Art

A conventional guide wire used in inserting a catheter to a blood vessel or a digestive organ is known (for example, Patent Literature 1). Patent Literature 1 discloses a technology of forming a bulged part in a distal end part of a guide wire, in the guide wire in which a coil spring is wound around outer circumference of a core.

### Citation List

### Patent Literature

Patent Literature 1: Japanese Patent Application Publication NO. 2-28246

### Summary of Invention

### Technical Problem

In general, when the diameter of the distal end of a guide wire is made large, while the backup property is improved so that the lesion passing property is improved, there is a fear that the guide wire punctures a blood vessel when advancing in the blood vessel. On the other hand, when the diameter of the distal end is made small, while blood vessel puncturing can be prevented, the backup property is degraded so that the lesion passing property is degraded as a problem. Furthermore, due to the degradation of the backup property, it is required that a support catheter or a micro catheter is used together when a guiding catheter having a large diameter is delivered to a target position in a blood vessel, so that a procedure becomes complicated as a problem. As described above, a technology for facilitating a procedure using a guide wire still has room for improvement.

The present invention has an object to provide a technology for facilitating a procedure using a guide wire.

### Solution to Problem

The present invention has been performed in order to solve at least part of the problem described above, and can be realized as aspects described below.

(1) According to an aspect of the present invention, a guide wire is provided. This guide wire includes: a core shaft; an engagement part provided in outer circumference of the core shaft; a cylindrical body provided in an outside of the core shaft and movable along an axis line direction of the core shaft; and an engaged part that is provided in the cylindrical body, and engages with the engagement part to regulate further movement of the cylindrical body in a first direction when the cylindrical body moves in the first direction along the axis line direction of the core shaft.
   According to this configuration, the rigidity in a distal end side of the guide wire can be changed by relatively moving the cylindrical body with respect to the core shaft. Thus, for example, when the guide wire advances in a blood vessel, blood vessel puncturing can be prevented by reducing the rigidity. Furthermore, at the time of lesion passing or when a guiding catheter having a large diameter is delivered to a target position in a blood vessel, the backup property can be improved by increasing the rigidity. According to this configuration, when the cylindrical body moves in the first direction along the axis line direction of the core shaft, the engagement part and the engaged part engage with each other to regulate further movement of the cylindrical body, so that the relative positions of the cylindrical body and the core shaft can be kept in an appropriate range. Accordingly, according to this configuration, a procedure using a guide wire can be facilitated.
(2) In the guide wire of the aspect described above, the engagement part may include a first engagement part, and a second engagement part provided in a proximal end of the core shaft from the first engagement part, and the engaged part may include a first engaged part that engages with the first engagement part to regulate further movement of the cylindrical body in the first direction when the cylindrical body moves in the first direction, and a second engaged part that engages with the second engagement part to regulate further movement of the cylindrical body in a second direction when the cylindrical body moves in the second direction along the axis line direction of the core shaft. According to this configuration, in either case where the cylindrical body moves in the first direction or the second direction along the axis line direction of the core shaft, the engagement part and the engaged part engage with each other to regulate further movement of the cylindrical body, so that the relative positions of the cylindrical body and the core shaft can be kept in an appropriate range more easily.
(3) In the guide wire according to the aspect described above, a length of the cylindrical body may be longer than a distance from the first engagement part to the second engagement part, and the cylindrical body may be movable along the axis line direction of the core shaft in a state of covering the first engagement part and the second engagement part. According to this configuration, the first engagement part and the second engagement part do not expose to an outer surface of the guide wire, so that the outer surface of the guide wire can be smooth. As a result, the guide wire can advance easily in the blood vessel.
(4) In the guide wire of the aspect described above, the first engagement part and the second engagement part may be annular projection parts formed along an outer circumferential direction of the core shaft. According to this configuration, the first engagement part and the second engagement part can easily engage with the first engaged part and the second engaged part, respectively, by the annular projection parts.
(5) In the guide wire of the aspect describe above, the first engaged part may be provided in a distal end of the cylindrical body, and the second engaged part may be provided in a proximal end of the cylindrical body. According to this configuration, the first engaged part and the second engaged part can be easily attached to the cylindrical body. When the first engagement part and the second engagement part are arranged in the inside of the first engaged part and the second engaged part, a degree of freedom of setting of a movable range of the cylindrical body can be increased.
(6) In the guide wire of the aspect described above, each of the first engaged part and the second engaged part may have an annular outer shape, a taper having an outer diameter expanding from a distal end side to a proximal end side may be formed in the first engaged part, and a taper having an outer diameter expanding from a proximal end side to a distal end side may be formed in the second engaged part. According to this configuration, a step between the core shaft and the cylindrical body can be smooth by the tapers. As a result, the guide wire can easily advance in the blood vessel.
(7) The guide wire of the aspect described above may further include a coil body covering a distal end side of the core shaft, and in the guide wire, the cylindrical body may cover a part of a proximal end side of the coil body, the first engagement part may be provided between a distal end and a proximal end of the coil body, and the first engaged part may be located in the distal end side of the core shaft from the first engagement part. According to this configuration, since the distal end side from the cylindrical body includes the core shaft and the coil body, the rigidity of the distal end part of the guide wire can be reduced. As a result, blood vessel puncturing by the distal end of the guide wire can further be prevented.
(8) In the guide wire of the aspect described above, the cylindrical body may be a coil body. According to this configuration, the rigidity and a torque transmission property of the guide wire can be improved.
(9) In the guide wire of the aspect described above, the cylindrical body may include an inner layer formed of a resin, a reinforcing layer arranged in an outer circumference of the inner layer, and an outer layer covering the reinforcing layer. According to this configuration, the inner layer can prevent a blood clot or the like from adhering to an inside of the cylindrical body. Furthermore, the outer layer can reduce a friction force due to contact of outer circumference of the cylindrical body and the lumen inner wall of a human. Furthermore, the reinforcing layer can reduce the thickness of the cylindrical body while securing the bending rigidity of the cylindrical body.
(10) In the guide wire of the aspect described above, the cylindrical body may include an inner layer formed of a resin, an intermediate coil body arranged in outer circumference of the inner layer, and an outer layer covering the intermediate coil body. According to this configuration, a blood clot or the like can be prevented from adhering to the inside of the cylindrical body, and a friction force due to contact of outer circumference of the cylindrical body and the lumen inner wall of a human can be reduced. Furthermore, the intermediate coil body can reduce the thickness of the cylindrical body while securing the bending rigidity of the cylindrical body.

Note that the present invention can be realized in various aspects, for example, aspects such as a manufacturing device of a guide wire or a manufacturing method of a guide wire.

### Brief Description of Drawings

Fig. 1 is an explanatory diagram illustrating an overall configuration of a guide wire of a first embodiment.
Fig. 2 is a diagram illustrating a state where a coil body is moved in a proximal end direction of a core shaft.
Fig. 3 is a diagram illustrating a state where the coil body is moved in a distal end direction of the core shaft.
Fig. 4 is an explanatory diagram illustrating a procedure using a guide wire of a comparative example.
Fig. 5 is an explanatory diagram illustrating a procedure using the guide wire of the present embodiment.
Fig. 6 is an explanatory diagram illustrating an overall configuration of a guide wire of a second embodiment.
Fig. 7 is an explanatory diagram illustrating an overall configuration of a guide wire of a third embodiment.
Fig. 8 is an explanatory diagram illustrating an overall configuration of a guide wire of a fourth embodiment.
Fig. 9 is an explanatory diagram illustrating an overall configuration of a guide wire of a fifth embodiment.
Fig. 10 is an explanatory diagram illustrating an overall configuration of a guide wire of a sixth embodiment.
Fig. 11 is an explanatory diagram illustrating an overall configuration of a guide wire of a seventh embodiment.
Fig. 12 is an explanatory diagram illustrating an overall configuration of a guide wire of an eighth embodiment.
Fig. 13 is an explanatory diagram illustrating an overall configuration of a guide wire of a ninth embodiment.
Fig. 14 is an explanatory diagram illustrating an overall configuration of a guide wire of a tenth embodiment.
Fig. 15 is an explanatory diagram illustrating an overall configuration of a guide wire of an eleventh embodiment.
Fig. 16 is an explanatory diagram illustrating an overall configuration of a guide wire of a twelfth embodiment.
Fig. 17 is an explanatory diagram illustrating an overall configuration of a guide wire of a thirteenth embodiment.
Fig. 18 is an explanatory diagram illustrating an overall configuration of a guide wire of a fourteenth embodiment.
Fig. 19 is an explanatory diagram illustrating an overall configuration of a guide wire of a fifteenth embodiment.
Fig. 20 is an explanatory diagram illustrating an overall configuration of a guide wire of a sixteenth embodiment.
Fig. 21 is an explanatory diagram illustrating an overall configuration of a guide wire of a seventeenth embodiment.
Fig. 22 is an explanatory diagram illustrating an overall configuration of a guide wire of an eighteenth embodiment.
Fig. 23 is a diagram illustrating a state where the cylindrical body is moved in the distal end direction of the core shaft.
Fig. 24 is an explanatory diagram illustrating flushing using a fluid in a syringe.
Fig. 25 is an explanatory diagram illustrating an overall configuration of a guide wire of a nineteenth embodiment.
Fig. 26 is a diagram illustrating a state where the cylindrical body is moved in the distal end direction of the core shaft.
Fig. 27 is an explanatory diagram illustrating a configuration of a proximal end side of a guide wire of a twentieth embodiment.
Fig. 28 is an explanatory diagram illustrating a configuration of a proximal end side of a guide wire of a twenty-first embodiment. Description of Embodiments

### <First Embodiment>

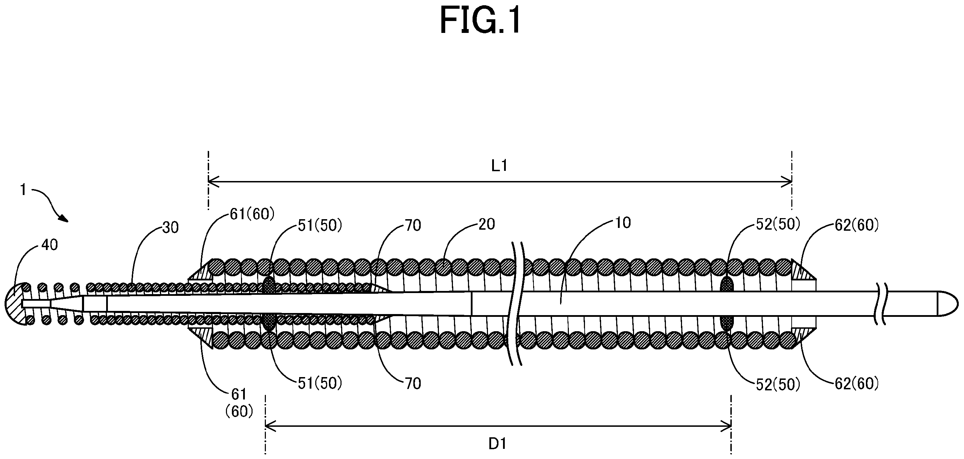

Fig. 1 is an explanatory diagram illustrating an overall configuration of a guide wire 1 of a first embodiment. The guide wire 1 is medical equipment used when a catheter is inserted to a blood vessel or a digestive organ, and includes a core shaft 10, a coil body 20, a distal end coil body 30, a distal end joint part 40, an engagement part 50, an engaged part 60, and an inner joint part 70. Hereinafter, a left side in Fig. 1 is referred to as a "distal end side" of the guide wire 1 and each component, and a right side in Fig. 1 is referred to as a "proximal end side" of the guide wire 1 and each component. The distal end side of the guide wire 1 is a side (distal side) of insertion to the body, and the proximal end side of the guide wire 1 is a side (proximal side) of operation by a technician such as a doctor.

The core shaft 10 is a long-shape member having an outer diameter that is configured to be smaller (tapered) from the proximal end side to the distal end side. The core shaft 10 can be formed of a material such as a stainless alloy (SUS302, SUS304, SUS316 or the like), a super-elastic alloy such as an Ni-Ti alloy, a piano wire, a nickel-chromium alloy, a cobalt alloy, or a tungsten. The core shaft 10 may be formed of a known material other than those described above. The length of the core shaft 10 is not limited particularly, but can be within a range from 1000 mm to 3000 mm, for example. Also the outer diameter of the core shaft 10 is not limited particularly, but can be within a range from 0.1 mm to 1.0 mm, for example. The distal end joint part 40 is formed in the distal end of the core shaft 10.

The distal end joint part 40 is formed by metal soldering of silver soldering, gold soldering, zinc, Sn-Ag alloy, Au-Sn alloy or the like, and this metal soldering fixes a distal end of the distal end coil body 30 and a distal end of the core shaft 10. Note that the distal end joint part 40 may be formed of an adhesive such as an epoxy adhesive, and the adhesive fixes the distal end of the distal end coil body 30 and the distal end of the core shaft 10.

The coil body 20 includes one of a plurality of coils, and is wound around the core shaft 10 so as to cover a part of the core shaft 10 and the distal end coil body 30. The coil body 20 is not fixed to the core shaft 10, and is configured to be relatively movable (slidable) with respect to the core shaft 10. That is, the coil body 20 is configured to be able to reciprocate along the axis line direction of the core shaft 10. Furthermore, the coil body 20 is configured to be relatively rotatable (coaxially rotatable) with respect to the core shaft 10. That is, the coil body 20 is configured to be rotatable with the axis line of the core shaft 10 as a rotation axis.

The coil included in the coil body 20 may be a single coil obtained by forming a cylindrical shape by spirally winding one strand having a circular cross section, or may be a hollow twisted wire coil obtained by forming a cylindrical shape by a twisted wire formed of a plurality of strands twisted. The coil body 20 may be formed by combining a single coil and a hollow twisted wire coil. The coil body 20 may be formed of a radiotranslucent alloy such as a stainless alloy (SUS302, SUS304, SUS316 or the like), a super-elastic alloy such as a Ni-Ti alloy, a piano wire, a nickel-chromium alloy, or a cobalt alloy, or a radiopaque alloy such as a gold, a platinum, a tungsten, or an alloy containing these elements (for example, a platinum-nickel alloy). The coil body 20 may be formed of a known material other than those described above. A length L1 of the coil body 20 is shorter than the length of the core shaft 10. The coil body 20 has a diameter configured to be constant. The outer diameter of the coil body 20 is not limited particularly, but can be within a range from 1.0 mm to 2.0 mm, for example.

The distal end coil body 30 is formed of a single coil or a hollow twisted wire coil, and is wound around the core shaft 10 so as to cover the outer circumference of the distal end side of the core shaft 10. Here, the distal end coil body 30 is wound around a part of a small diameter part and a taper part in the distal end side of the core shaft 10. The length of the distal end coil body 30 is shorter than the length of the core shaft 10 and the length of the coil body 20. The length of the distal end coil body 30 is not limited particularly, but can be within a range from 10 mm to 100 mm, for example. The inner diameter of the distal end coil body 30 is larger than the outer diameter of the core shaft 10, and the outer diameter of the distal end coil body 30 is smaller than the inner diameter of the coil body 20. The outer diameter of the distal end coil body 30 is formed to be constant from the distal end to the proximal end. The outer diameter of the distal end coil body 30 is not limited particularly, but can be within a range from 0.1 mm to 1.0 mm, for example.

The distal end of the distal end coil body 30 is jointed to the distal end of the core shaft 10 by the distal end joint part 40. The proximal end of the distal end coil body 30 is jointed to the taper part of the core shaft 10 by the inner joint part 70. The distal end coil body 30 is fixed to the core shaft 10 by the distal end joint part 40 and the inner joint part 70. The inner joint part 70 may be formed of the same material as or a different material from the material of the distal end joint part 40. The winding direction of the distal end coil body 30 may be the same as or different from the winding direction of the coil body 20. The coil pitch of the distal end coil body 30 may be the same as or different from the coil pitch of the coil body 20. Note that the distal end coil body 30 has a sparsely wound part and a densely wound part having different coil pitches from each other, and the sparsely wound part is formed in the distal end side, and the densely wound part is formed in the proximal end side.

The engagement part 50 is a stopper that is provided in the outer circumference of the core shaft 10 and engages with the engaged part 60 of the coil body 20, and includes a distal end side engagement part 51 and a proximal end side engagement part 52. Each of the distal end side engagement part 51 and the proximal end side engagement part 52 is an annular-shape (ring-shape) member fixed to the outer circumference of the core shaft 10, in other words, an annular projection part formed along the outer circumferential direction of the core shaft 10. The distal end side engagement part 51 and the proximal end side engagement part 52 may be formed of the same material as the material of the distal end joint part 40, and may be formed of a different material as the material of the distal end joint part 40. The outer diameter of the distal end side engagement part 51 and the proximal end side engagement part 52 is larger than the outer diameter of the distal end coil body 30 and the outer diameter of the core shaft 10, and is smaller than the inner diameter of the coil body 20. The distal end side engagement part 51 and the proximal end side engagement part 52 are arranged in positions in the inside of the coil body 20 in the core shaft 10.

The distal end side engagement part 51 is formed in the distal end side of the core shaft 10 from the proximal end side engagement part 52, and here, the distal end side engagement part 51 is formed in a position covered with the distal end coil body 30 in the core shaft 10. In other words, the proximal end side engagement part 52 is provided between the distal end and the proximal end of the distal end coil body 30. The distal end side engagement part 51 and the proximal end side engagement part 52 engage with a distal end side engaged part 61 and a proximal end side engaged part 62 as described later, to regulate movement of the coil body 20 in the axis line direction. Here, when the distance from the distal end side engagement part 51 to the proximal end side engagement part 52 is a distance D1, the distance D1 is shorter than the length L1 of the coil body 20.

The engaged part 60 is a stopper that is provided in the coil body 20, and engages with the engagement part 50 of the core shaft 10, and includes the distal end side engaged part 61 and the proximal end side engaged part 62. The distal end side engaged part 61 and the proximal end side engaged part 62 are annular-shape (ring-shape) members jointed to both ends of the coil body 20, and are formed of the same material as the material of the distal end joint part 40. The inner diameter of each of the distal end side engaged part 61 and the proximal end side engaged part 62 is configured to be smaller than the inner diameter of the coil body 20. As a result, the inner circumferential surfaces of the distal end side engaged part 61 and the proximal end side engaged part 62 project further in the inside than the inner circumferential surface of the coil body 20.

The distal end side engagement part 61 is jointed to the distal end of the coil body 20, and is located in the distal end side from the distal end side engagement part 51 provided in the core shaft 10. The inner diameter of the distal end side engaged part 61 is smaller than the outer diameter of the distal end side engagement part 51 provided in the core shaft 10. As a result, when the coil body 20 relatively moves in the proximal end direction (right direction in Fig. 1) with respect to the core shaft 10, the distal end side engaged part 61 engages (contacts) with the distal end side engagement part 51. A taper is formed in the outside of the distal end side engaged part 61, and the distal end side engaged part 61 is configured so that the outer diameter expands from the distal end side to the proximal end side. As a result, in the guide wire 1, a step between the outer diameter of the distal end coil body 30 and the outer diameter of the coil body 20 can be smooth.

The proximal end side engaged part 62 is jointed to the proximal end of the coil body 20, and is located in the proximal end side from the proximal end side engagement part 52 provided in the core shaft 10. The inner diameter of the proximal end side engaged part 62 is smaller than the outer diameter of the proximal side engagement part 52 provided in the core shaft 10. As a result, when the coil body 20 relatively moves in the distal end direction (left direction in Fig. 1) with respect to the core shaft 10, the proximal end side engaged part 62 engages (contacts) with the proximal end side engagement part 52. A taper is formed in the outside of the proximal end side engaged part 62, and the proximal end side engaged part 62 is configured so that the outer diameter expands from the proximal end side to the distal end side. As a result, in the guide wire 1, a step between the outer diameter of the coil body 20 and the outer diameter of the core shaft 10 can be smooth.

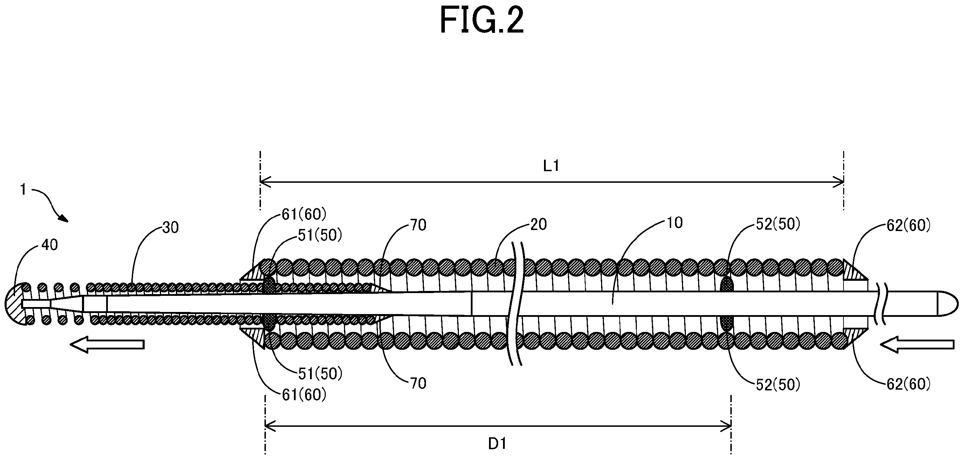

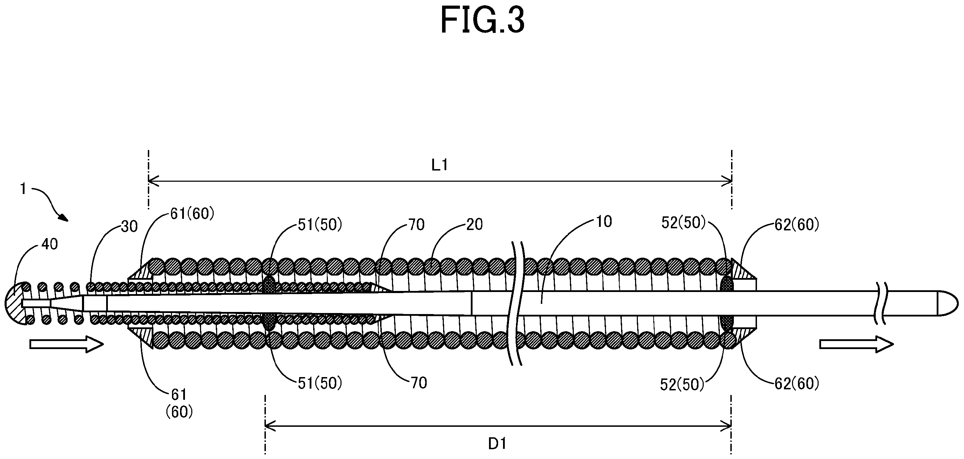

The relative movement of the coil body 20 and the core shaft 10 will be described with reference to Figs. 2 and 3. Fig. 2 is an explanatory diagram illustrating a state where the coil body 20 is relatively moved in the proximal end direction (proximal end side) of the core shaft 10. Fig. 3 is an explanatory diagram illustrating a state where the coil body 20 is relatively moved in the distal end direction (distal end side) of the core shaft 10. As described already, the length L1 of the coil body 20 is longer than the distance D1 from the distal end side engagement part 51 to the proximal end side engagement part 52, and the coil body 20 is configured to be able to reciprocate along the axis line direction of the core shaft 10 in a range from a position shown in Fig. 2 to a position shown in Fig. 3 in a state of covering the distal end side engagement part 51 and the proximal end side engagement part 52. That is, in the guide wire 1, the length (projection amount) of the small diameter portion including the distal end coil body 30 projecting from the distal end of the coil body 20 and the distal end joint part 40 can be changed without changing the position of the coil body 20, and this small diameter portion can be relatively rotated with respect to the coil body 20.

As shown in Fig. 2, when the coil body 20 is relatively moved in the proximal end direction of the core shaft 10, the distal end side engaged part 61 contacts the distal end side engagement part 51 to regulate further movement of the coil body 20 in the proximal end direction. At this time, the length (projection amount) of the small diameter portion including the distal end coil body 30 projecting from the distal end of the coil body 20 and the distal end joint part 40 becomes the maximum. The projection amount at this time is set arbitrary, but can be within a range from 50 to 200 mm, for example. On the other hand, as shown in Fig. 3, when the coil body 20 is relatively moved in the distal end direction of the core shaft 10, the proximal end side engaged part 62 contacts the proximal end side engaged part 52 to regulate further movement of the coil body 20 in the distal end direction. At this time, the length (projection amount) of the small diameter portion including the distal end coil body 30 projecting from the distal end of the coil body 20 and the distal end joint part 40 becomes the minimum. The projection amount at this time is set arbitrary, but can be within a range from 10 to 100 mm, for example.

### <Example of Effect of Present Embodiment>

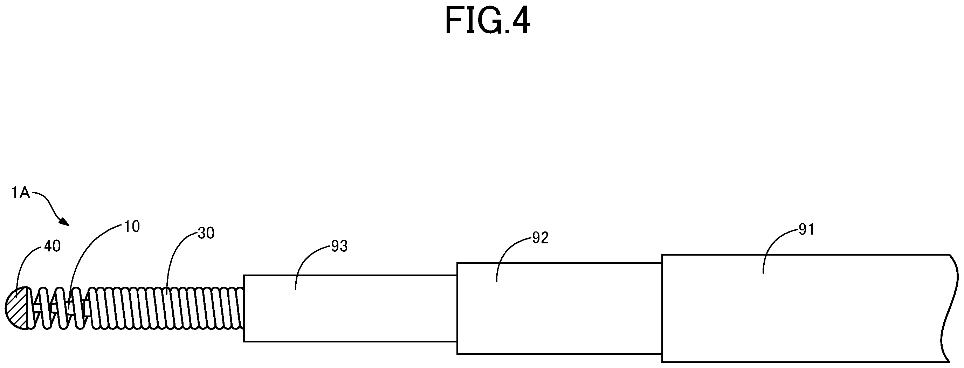

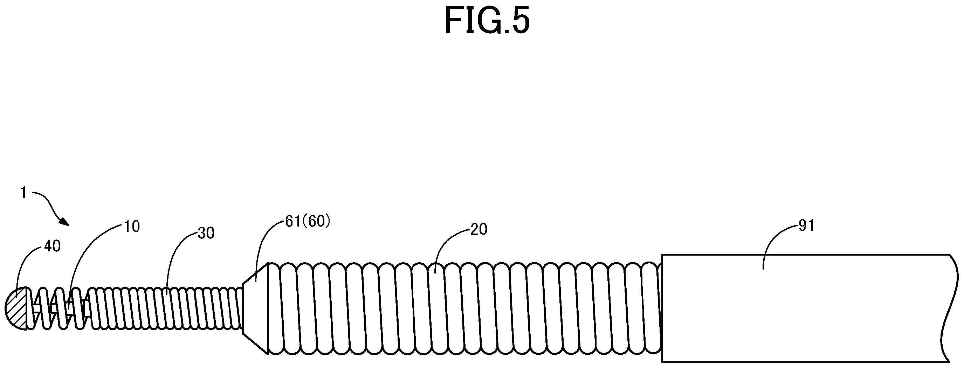

An example of the effect of the present embodiment will be described with reference to Figs. 4 and 5. Fig. 4 is an explanatory diagram illustrating a procedure using a guide wire 1A of a comparative example. Fig. 5 is an explanatory diagram illustrating a procedure using the guide wire 1 of the present embodiment. The difference of the guide wire 1A of the comparative example shown in Fig. 4 from the guide wire 1 of the present embodiment is that the guide wire 1A does not include the coil body 20. That is, the guide wire 1A of the comparative example has a configuration in which the distal end coil body 30 is arranged in the distal end of the core shaft 10, and the core shaft 10 is exposed in the proximal end side of the core shaft 10. For example, in stroke treatment (cerebrovascular region), for example, when a guiding catheter 91 having a large diameter of 6 Fr to 7 Fr is delivered to a target blood vessel using the guide wire 1A of the comparative example having the outer diameter of about 0.014 inch (0.36 mm), the support property of the guide wire 1A is insufficient, so that the guiding catheter 91 cannot be delivered to the target blood vessel only with the guide wire 1A. Therefore, As shown in Fig. 4, it is required that the guiding catheter is delivered to the target blood vessel using a support catheter 92 and a micro catheter 93 together. Note that, when the diameter of the core shaft 10 and the distal end coil body 30 of the distal end of the guide wire 1A of the comparative example is made large, the rigidity of the distal end is increased, so that while the support catheter 92 and the micro catheter 93 become unnecessary, there is a fear that the blood vessel is punctured when the guide wire advances in the blood vessel. Particularly, there is fear that the blood vessel is punctured when the guide wire advances in a blood vessel that is not shown in a screen under X-ray fluoroscopy due to a blood clot.

The guide wire 1 of the present embodiment has two types of outer diameters of the distal end coil body 30 and the coil body 20, and has a configuration in which a guide wire having a large diameter and a guide wire having a small diameter are integrated. Since the distal end of the guide wire 1 is formed of the distal end coil body 30 having a small diameter, puncturing when the guide wire 1 advances in a blood vessel to select a target blood vessel can be reduced. Since the guide wire 1 includes the coil body 20 having a large diameter in the proximal end side of the distal end coil body 30, the guide wire 1 has rigidity for delivering the guiding catheter 91 to a target blood vessel only with the guide wire 1 without using the support catheter 92 and the micro catheter 93. Thus, according to the guide wire 1 of the present embodiment, since the guiding catheter 91 can be delivered to the target blood vessel without using a support catheter and a micro catheter, costs required for the support catheter and the micro catheter can be reduced.

According to the guide wire 1 of the present embodiment described above, as shown in Figs. 2 and 3, by relatively moving the coil body 20 with respect to the core shaft 10, the rigidity of the distal end side of the guide wire 1 can be changed. That is, according to the guide wire 1 of the present embodiment, the rigidity of the guide wire distal end part can be adjusted by a technician. Specifically, as shown in Fig. 2, by increasing the length of the small diameter portion formed of the distal end coil body 30 projecting from the distal end of the coil body 20 and the core shaft 10, the rigidity of the distal end side of the guide wire 1 can be reduced. As a result, for example, when the guide wire advances in a blood vessel, blood vessel puncturing can be prevented. On the other hand, as shown in Fig. 3, by decreasing the length of the small diameter portion formed of the distal end coil body 30 projecting from the distal end of the coil body 20 and the core shaft 10, and setting the coil body 20 having a large diameter in the distal end side, the rigidity of the distal end side of the guide wire 1 can be increased. As a result, for example, at the time of lesion passing or when the guiding catheter 91 having a large diameter is delivered to a target blood vessel, the backup property can be improved.

According to the guide wire 1 of the present embodiment, as shown in Fig. 2, when the coil body 20 is moved in the proximal end direction of the core shaft 10 along the axis line direction of the core shaft 10, the distal end side engaged part 61 contacts the distal end side engagement part 51 to regulate further movement of the coil body 20 in the proximal end direction. As shown in Fig. 3, when the coil body 20 is moved in the distal end direction of the core shaft 10 along the axis line direction of the core shaft 10, the proximal end side engaged part 62 contacts the proximal end side engagement part 52 to regulate further movement of the coil body 20 in the distal end direction. As a result, the relative positions of the guide wire 1 and the core shaft can be kept in an appropriate range, and the procedure using the guide wire 1 can be facilitated.

According to the guide wire 1 of the present embodiment, the distal end side engagement part 51 and the proximal end side engagement part 52 are arranged in a region located in the inside of the coil body 20 in the core shaft 10, so that the distal end side engagement part 51 and the proximal end side engagement part 52 are not exposed to the outer surface of the guide wire 1. As a result, the outer surface of the guide wire 1 can be smooth. As a result, the guide wire can easily advance in a blood vessel (insertion property and operation property with a device used together can be improved).

According to the guide wire 1 of the present embodiment, each of the distal end side engagement part 51 and the proximal end side engagement part 52 is configured as an annular projection part. As a result, the distal end side engagement part 51 and the proximal end side engagement part 52 can easily engage with the distal end side engaged part 61 and the proximal end side engaged part 62. According to the guide wire 1 of the present embodiment, the distal end side engaged part 61 and the proximal end side engaged part 62 are provided in both end portions of the coil body 20, so that the distal end side engaged part 61 and the proximal end side engaged part 62 can be easily jointed to the coil body 20. Since the distal end side engaged part 61 and the proximal end side engaged part 62 are provided in both end portions of the coil body 20, the movement range of the coil body 20 from when one of the distal end side engaged part 61 and the proximal end side engaged part 62 engages until the other of them engages can be set relatively widely. As a result, the degree of freedom of setting of the movable range of the coil body 20 can be increased. According to the guide wire 1 of the present embodiment, each of the distal end side engaged part 61 and the proximal end side engaged part 62 has an annular outer shape, and has a taper formed. By this taper, the step between the core shaft and the cylindrical body can be smooth, and the guide can easily advance in a blood vessel.

According to the guide wire 1 of the present embodiment, since the diameter of the core shaft 10 is reduced in the distal end portion of the guide wire 1, and the distal end coil body 30 is arranged in the outside, the rigidity of the distal end side from the cylindrical body can be reduced. As a result, blood vessel puncturing due to the distal end of the guide wire can further be prevented.

### <Second Embodiment>

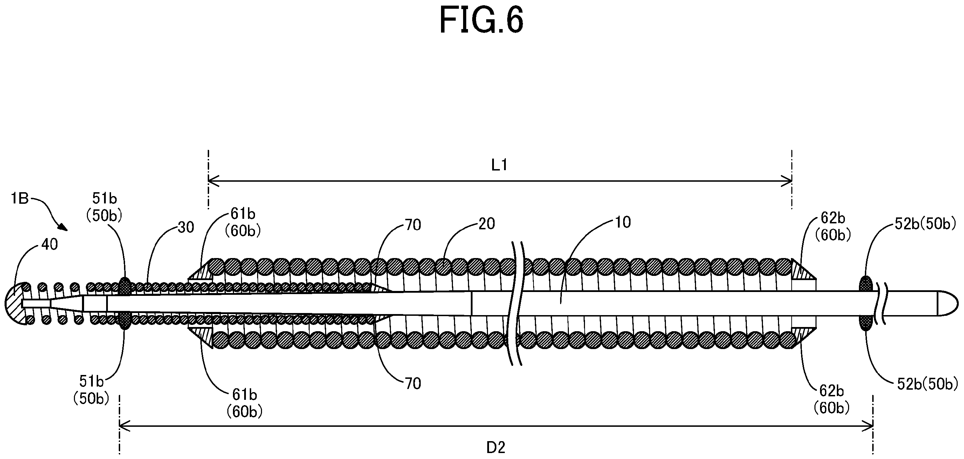

Fig. 6 is an explanatory diagram illustrating an overall configuration of a guide wire 1B of a second embodiment. A difference of the guide wire 1B of the second embodiment from the guide wire 1 of the first embodiment is the positions of the distal end side engagement part 51 and the proximal end side engaged part 62 in the core shaft 10. Since other configurations are similar to those of the guide wire 1 of the first embodiment, description thereof is omitted. In the guide wire 1B of the second embodiment, a distal end side engagement part 51b is located in the distal end side of the core shaft 10 from the distal end side engaged part 61 of the coil body 20, and the proximal end side engagement part 52 is located in the proximal end side from the proximal end side engaged part 62 of the coil body 20. The shapes of the distal end side engagement part 51b and a proximal end side engagement part 52b may be the same as or different from the shapes of the distal end side engagement part 51 and the proximal end side engagement part 52 of the first embodiment.

In the guide wire 1B of the second embodiment, a distance D2 from the distal end side engagement part 51b to the proximal end side engagement part 52b is longer than the length L1 of the coil body 20. The coil body 20 is configured to be able to reciprocate between the distal end side engagement part 51b and the proximal end side engagement part 52b along the axis line direction of the core shaft 10. When the coil body 20 is relatively moved in the proximal end direction of the core shaft 10, a proximal end side engaged part 62b of the core shaft 10 contacts the proximal end side engagement part 52b to regulate further movement of the coil body 20 in the proximal end direction. On the other hand, when the coil body 20 is relatively moved in the distal end direction of the core shaft 10, a distal end side engaged part 61b of the coil body 20 contacts the distal end side engagement part 51b to regulate further movement of the coil body 20 in the distal end direction. The shapes of the distal end side engaged part 61b and the proximal end side engaged part 62b may be the same as or different from the shapes of the distal end side engaged part 61 and the proximal end side engaged part 62 of the first embodiment.

According to the guide wire 1B of the present embodiment described above, the distal end side engagement part 51b and the proximal end side engagement part 52b may be arranged in the outside of the distal end side engaged part 61b and the proximal end side engaged part 62b of the coil body 20. That is, the distal end side engagement part 51b and the proximal end side engagement part 52b may not be arranged in the inside of the coil body 20. Even in this case, by relatively moving the coil body 20 with respect to the core shaft 10, the rigidity of the distal end side of the guide wire 1B can be changed.

### <Third Embodiment>

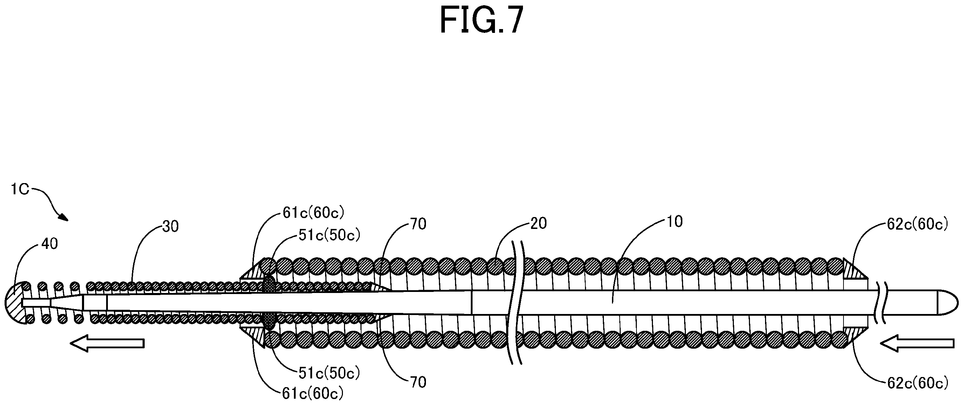

Fig. 7 is an explanatory diagram illustrating an overall configuration of a guide wire 1C of a third embodiment. A difference of the guide wire 1C of the third embodiment from the guide wire 1 of the first embodiment is that the core shaft 10 does not include the proximal end side engagement part 52. Since other configurations are similar to those of the guide wire 1 of the first embodiment, description thereof is omitted. In the guide wire 1C of the third embodiment, when the coil body 20 is relatively moved in the proximal end direction of the core shaft 10, as similar to the first embodiment, the distal end side engaged part 61c contacts a distal end side engagement part 51c to regulate further movement of the coil body 20 in the proximal end direction. On the other hand, when the coil body 20 is relatively moved in the distal end direction of the core shaft 10, the movement of the coil body 20 in the distal end direction is not regulated until a proximal end side engaged part 62c contacts the distal end side engagement part 51c. The shapes of a distal end side engaged part 61c, the proximal end side engaged part 62c, and the distal end side engagement part 51c may be the same as or different from the shapes of the distal end side engaged part 61, the proximal end side engaged part 62, and the distal end side engagement part 51 of the first embodiment.

According to the guide wire 1C of the present embodiment described above, the guide wire 1C may not include the proximal end side engagement part 52. Even in this case, when the coil body 20 moves in the proximal end direction of the core shaft 10, the distal end side engaged part 61c contacts the distal end side engagement part 51c to regulate further movement of the coil body 20 in the proximal end direction, so that the relative positions of the guide wire 1 and the core shaft can be kept in an appropriate range. As a result, the procedure using the guide wire 1 can be facilitated.

### <Fourth Embodiment>

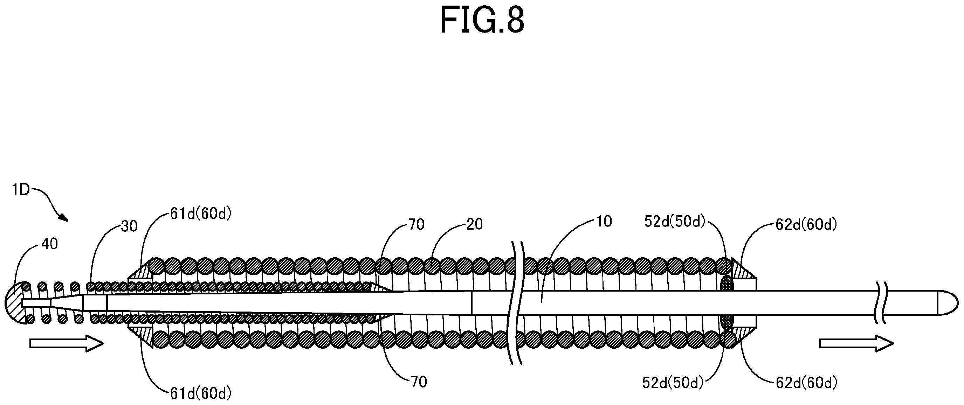

Fig. 8 is an explanatory diagram illustrating an overall configuration of a guide wire ID of a fourth embodiment. A difference of the guide wire ID of the fourth embodiment from the guide wire 1 of the first embodiment is that the core shaft 10 does not include the distal end side engagement part 51. Since other configurations are similar to those of the guide wire 1 of the first embodiment, description thereof is omitted. In the guide wire ID of the fourth embodiment, when the coil body 20 is relatively moved in the distal end direction of the core shaft 10, as similar to the first embodiment, a proximal end side engaged part 62d contacts a proximal end side engagement part 52d to regulate further movement of the coil body 20 in the distal end direction. On the other hand, when the coil body 20 is relatively moved in the proximal end direction of the core shaft 10, the movement of the coil body 20 in the proximal end direction is not regulated until a distal end side engaged part 61d contacts the proximal end side engagement part 52d. The shapes of the distal end side engaged part 61d, the proximal end side engaged part 62d, and the proximal end side engagement part 52d may be the same as or different from the shapes of the distal end side engaged part 61, the proximal end side engaged part 62, and the proximal end side engagement part 52 of the first embodiment.

According to the guide wire ID of the present embodiment described above, the guide wire ID may not include the distal end side engagement part 51. Even in this case, when the coil body 20 moves in the distal end direction of the core shaft 10, the proximal end side engaged part 62 contacts the proximal end side engagement part 52 to regulate further movement of the coil body 20 in the distal end direction, so that the relative positions of the guide wire 1 and the core shaft can be kept in an appropriate range. As a result, the procedure using the guide wire 1 can be facilitated.

### <Fifth Embodiment>

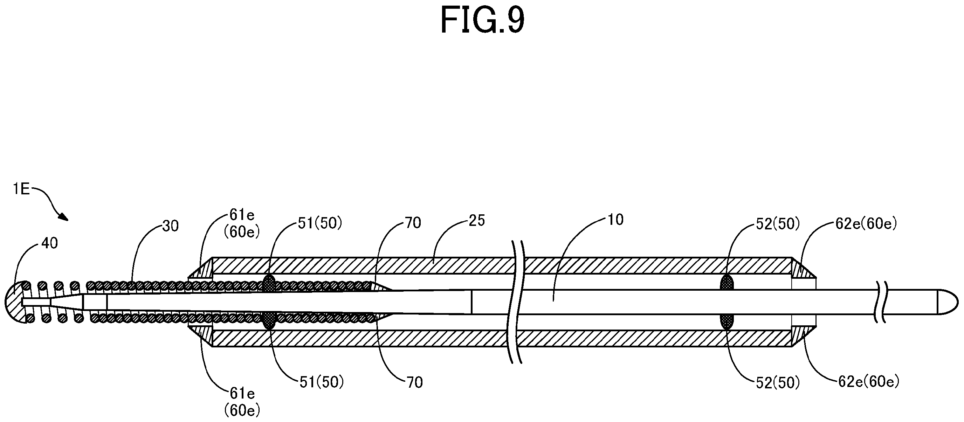

Fig. 9 is an explanatory diagram illustrating an overall configuration of a guide wire 1E of a fifth embodiment. A difference of the guide wire 1E of the fifth embodiment from the guide wire 1 of the first embodiment is that the cylindrical body 25 is provided instead of the coil body 20. Since other configurations are similar to those of the guide wire 1 of the first embodiment, description thereof is omitted. The cylindrical body 25 is a tube formed of a resin, and has the core shaft 10 arranged inside thereof. The resin material forming the cylindrical body 25 is not limited particularly, but can be, for example, polytetrafluoroethylene (PTFE), polyvinylidene difluoride (PVDF), perfluoro alkoxyl alkane (PFA), fluorinated ethylene propylene (FEP), ethylene tetrafluoro ethylene (ETFE), polyethylene (PE), or polypropylene (PP). As similar to the coil body 20 of the first embodiment, the cylindrical body 25 is not fixed to the core shaft 10, and is configured to be able to reciprocate along the axis line direction of the core shaft 10. The cylindrical body 25 is configured to be relatively rotatable (coaxially rotatable) with respect to the core shaft 10. A distal end side engaged part 61e and a proximal end side engaged part 62e are attached to both ends of the cylindrical body 25. The shapes of the distal end side engaged part 61e and the proximal end side engaged part 62e may be the same as or different from the shapes of the distal end side engaged part 61 and the proximal end side engaged part 62 of the first embodiment.

As the guide wire 1E of the present embodiment described above, the guide wire may not include the coil body 20. That is, the cylindrical body forming the large diameter part of the guide wire is not limited to the coil body 20. Even with a resin tube as the cylindrical body 25 of the present embodiment, by relatively moving this cylindrical body 25 with respect to the core shaft 10, the rigidity of the distal end side of the guide wire 1E can be changed.

### <Sixth Embodiment>

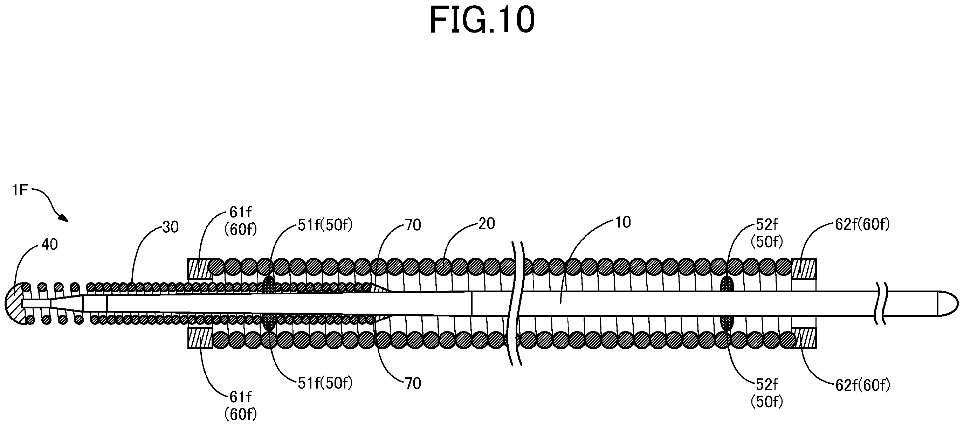

Fig. 10 is an explanatory diagram illustrating an overall configuration of a guide wire IF of a sixth embodiment. A difference of the guide wire IF of the sixth embodiment from the guide wire 1 of the first embodiment is the shapes of the distal end side engaged part 61 and the proximal end side engaged part 62. Since other configurations are similar to those of the guide wire 1 of the first embodiment, description thereof is omitted. Both of a distal end side engaged part 61f and a proximal end side engaged part 62f of the sixth embodiment do not include a taper. For other portions, they are the same as the distal end side engaged part 61 and the proximal end side engaged part 62 of the first embodiment. Even in this case, when the coil body 20 is relatively moved in the proximal end direction of the core shaft 10, the proximal end side engaged part 62f of the core shaft 10 contacts a proximal end side engagement part 52f to regulate further movement of the coil body 20 in the proximal end direction. On the other hand, when the coil body 20 is relatively moved in the distal end direction of the core shaft 10, the distal end side engaged part 61f of the coil body 20 contacts a distal end side engagement part 51f to regulate further movement of the coil body 20 in the distal end direction. The shapes of the distal end side engaged part 61f and the distal end side engagement part 51f may be arbitrary shapes as long as they can engage with each other. The shapes of the proximal end side engaged part 62f and the proximal end side engagement part 52f may be arbitrary shapes as long as they can engage with each other.

Also according to the guide wire IF of the present embodiment described above, by relatively moving the coil body 20 with respect to the core shaft 10, the rigidity of the distal end side of the guide wire IF can be changed. Note that the step between the core shaft 10 and the coil body 20 can be smoother when the distal end side engaged part 61 and the proximal end side engaged part 62 have a taper, and this is more preferable since the guide wire can easily advance in a blood vessel.

### <Seventh Embodiment>

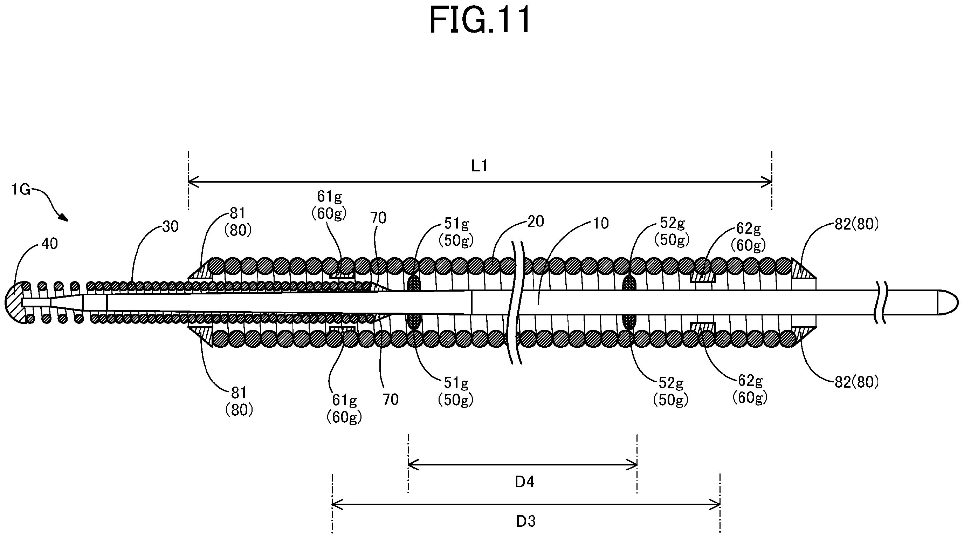

Fig. 11 is an explanatory diagram illustrating an overall configuration of a guide wire 1G of a seventh embodiment. A difference of the guide wire 1G of the seventh embodiment from the guide wire 1 of the first embodiment is the position and shape of each of the distal end side engaged part 61 and the proximal end side engaged part 62, and the position of each of the distal end side engagement part 51 and the proximal end side engagement part 52. Since other configurations are similar to those of the guide wire 1 of the first embodiment, description thereof is omitted. A distal end side engaged part 61g and a proximal end side engaged part 62g of the seventh embodiment are fixed not to both ends of the coil body 20 but to the inside of the coil body 20. That is, a distance D3 from the distal end side engaged part 61g to the proximal end side engaged part 62g of the seventh embodiment is shorter than the length L1 of the coil body 20. The distal end side engaged part 61g and the proximal end side engaged part 62g are annular-shape (ring-shape) members jointed to the inside of the coil body 20, and the distal end side engaged part 61g is arranged in the distal end side relatively from the proximal end side engaged part 62g. Since the inner diameter of each of the distal end side engaged part 61g and the proximal end side engaged part 62g is smaller than the inner diameter of the coil body 20, the distal end side engaged part 61g and the proximal end side engaged part 62g project to the inside of the coil body 20. A distal end forming part 81 and a proximal end forming part 82 are jointed to the both ends of the coil body 20. The distal end forming part 81 and the proximal end forming part 82 are not necessary to have an engaged part, and can be made to arbitrary shapes.

Each of the distal end side engagement part 51g and the proximal end side engagement part 52g of the seventh embodiment is formed between the distal end side engaged part 61g and the proximal end side engaged part 62g. A distance D4 from the distal end side engagement part 51g to the proximal end side engagement part 52g is smaller than the distance D1 from the distal end side engagement part 51 to the proximal end side engagement part 52 of the first embodiment. The shapes of the distal end side engagement part 51g and the proximal end side engagement part 52g may be the same as or different from the shapes of the distal end side engagement part 51 and the proximal end side engagement part 52 of the first embodiment. That is, the shapes of the distal end side engaged part 61g and the distal end side engagement part 51g may be arbitrary shapes as long as they can engage with each other. The shapes of the proximal end side engaged part 62g and the proximal end side engagement part 52g may be arbitrary shapes as long as they can engage with each other.

Since the distal end side engaged part 61g and the proximal end side engaged part 62g of the seventh embodiment project to the inside of the coil body 20, when the coil body 20 is relatively moved in the proximal end direction of the core shaft 10, the distal end side engaged part 61g engages with the distal end side engagement part 51g. When the coil body 20 is relatively moved in the distal end direction of the core shaft 10, the proximal end side engaged part 62g engages with the proximal end side engagement part 52g.

According to the guide wire 1G of the present embodiment described above, the positions of the distal end side engaged part 61 and the proximal end side engaged part 62 are not limited both ends of the coil body 20. As the guide wire 1G of the present embodiment, the distal end side engaged part 61g and the proximal end side engaged part 62g can be fixed to arbitrary positions in the inside of the coil body 20. Even in this case, the relative positions of the guide wire 1G and the core shaft 10 can be kept within an appropriate range, and the procedure using the guide wire 1G can be facilitated.

### <Eighth Embodiment>

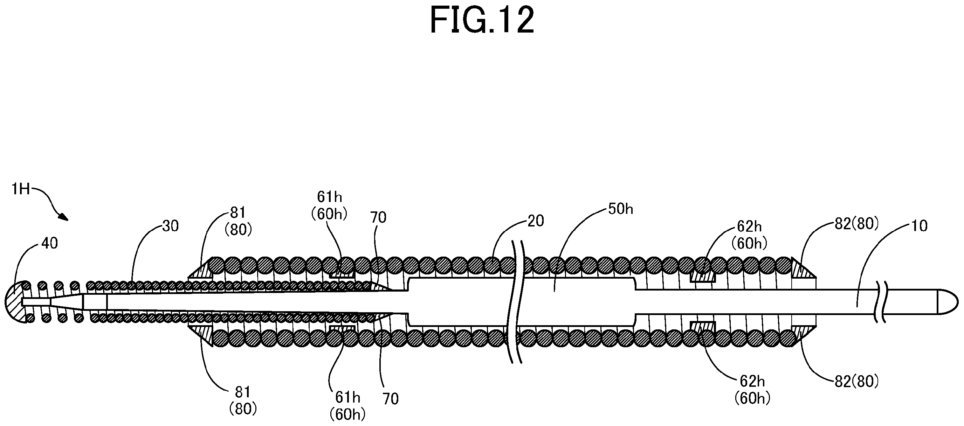

Fig. 12 is an explanatory diagram illustrating an overall configuration of a guide wire 1H of an eighth embodiment. A difference of the guide wire 1H of the eighth embodiment from the guide wire 1 of the first embodiment is positions and shapes of the engagement part 50 and the engaged part 60. Since other configurations are similar to those of the guide wire 1 of the first embodiment, description thereof is omitted. Unlike the first embodiment, in an engagement part 50h of the eighth embodiment, one projection part contacts both the distal end side engaged part 61h and the proximal end side engaged part 62h. Here, in the engagement part 50h, the diameter of a portion of the core shaft 10 is increased in a predetermined section, the distal end side of this diameter increasing section contacts the distal end side engaged part 61h, and the proximal end side of the diameter increasing section contacts the proximal end side engaged part 62h. Each of the distal end side engaged part 61h and the proximal end side engaged part 62h is fixed not to both ends of the coil body 20, but to the inside of the coil body 20. The distal end side engaged part 61h and the proximal end side engaged part 62h are annular-shape (ring-shape) members jointed to the inside of the coil body 20, and each of them projects to the inside of the coil body 20. The distal end forming part 81 and the proximal end forming part 82 are jointed to both ends of the coil body 20. The distal end forming part 81 and the proximal end forming part 82 do not necessarily have an engaged part, and may have arbitrary shapes.

According to the guide wire 1H of the present embodiment described above, the engagement part 50h does not necessarily include a projection part that contacts the distal end side engaged part 61h and a projection part that contacts the proximal end side engaged part 62 separately from each other, and one projection part that contacts with both of them may be configured. The engagement part 50 is not necessarily formed as a separate body from the core shaft 10, and may be formed as a portion of the core shaft 10.

### <Ninth Embodiment>

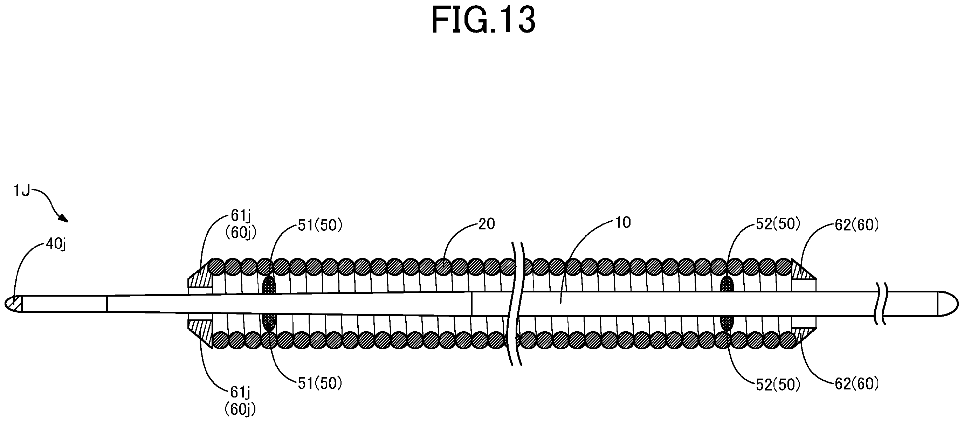

Fig. 13 is an explanatory diagram illustrating an overall configuration of a guide wire 1J of a ninth embodiment. A difference of the guide wire 1J of the ninth embodiment from the guide wire 1 of the first embodiment is that the guide wire 1J does not include the distal end coil body 30. Since other configurations are similar to the guide wire 1 of the first embodiment, description thereof is omitted. In the guide wire 1J of the ninth embodiment, by relatively moving the coil body 20 with respect to the core shaft 10, the length of the core shaft 10 projecting from the distal end of the coil body 20 can be changed.

According to the guide wire 1J of the present embodiment described above, by relatively moving the coil body 20 with respect to the core shaft 10, the rigidity of the distal end side of the guide wire 1 can be changed. Specifically, by increasing the length of the core shaft 10 as a small diameter portion projecting from the distal end of the coil body 20, the rigidity of the distal end side of the guide wire 1 can be reduced. On the other hand, by reducing the length of the core shaft 10 projecting from the distal end of the coil body 20, and setting the coil body 20 having a large diameter in the distal end side, the rigidity of the distal end side of the guide wire 1 can be improved.

### <Tenth Embodiment>

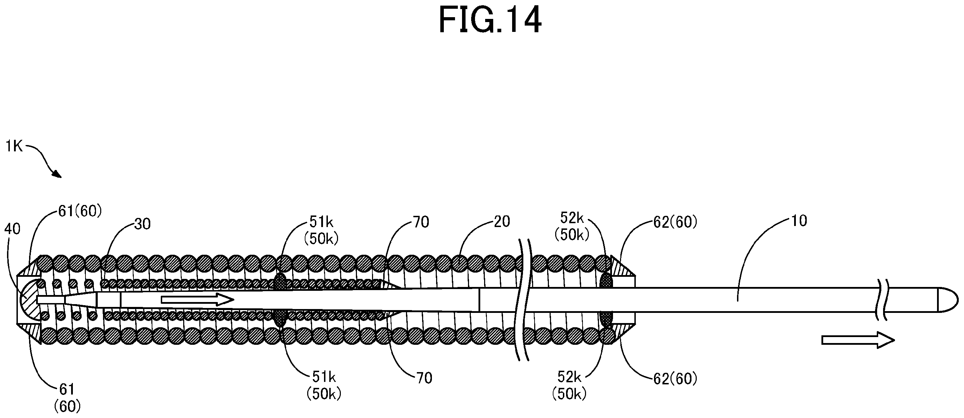

Fig. 14 is an explanatory diagram illustrating an overall configuration of a guide wire 1K of a tenth embodiment. A difference of the guide wire 1K of the tenth embodiment from the guide wire 1 of the first embodiment is the movement range of the coil body 20 with respect to the core shaft 10. Since other configurations are similar to those of the guide wire 1 of the first embodiment, description thereof is omitted. In the guide wire 1K of the tenth embodiment, a proximal end side engagement part 52k is arranged in the distal end side from the proximal end side engagement part 52 of the first embodiment. Therefore, when the coil body 20 is moved in the distal end direction of the core shaft 10, and the proximal end side engaged part 62 contacts the proximal end side engagement part 52k, the distal end of the coil body 20 is located in the distal end side from the distal end joint part 40. That is, when the proximal end side engaged part 62 contacts the proximal end side engagement part 52k, the small diameter portion including the distal end coil body 30 and the core shaft 10 does not project from the distal end side of the coil body 20.

Even in this case, by relatively moving the coil body 20 with respect to the core shaft 10 to increase the length of the small diameter portion projecting from the distal end of the coil body 20, the rigidity of the distal end side of the guide wire 1 can be reduced. On the other hand, by reducing the length of the small diameter portion projecting from the distal end of the coil body 20, or making the small diameter portion not to project to set the coil body 20 having a large diameter in the distal end side, the rigidity of the distal end side of the guide wire 1 can be improved.

### <Eleventh Embodiment>

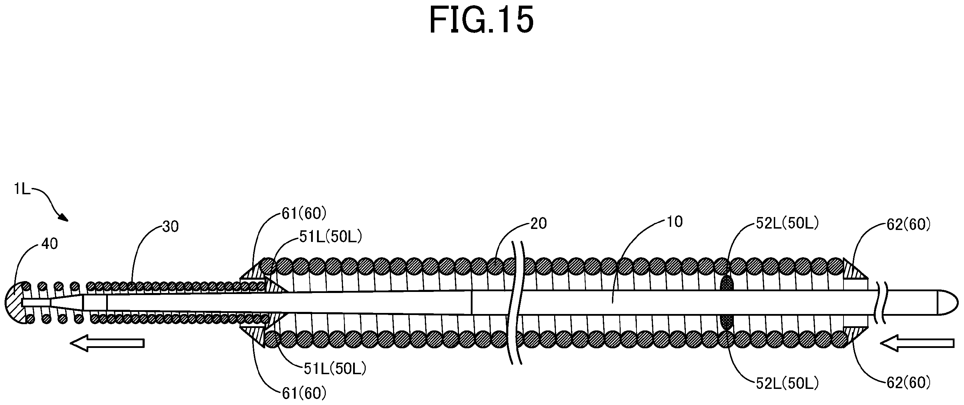

Fig. 15 is an explanatory diagram illustrating an overall configuration of a guide wire 1L of an eleventh embodiment. A difference of the guide wire 1L of the eleventh embodiment from the guide wire 1 of the first embodiment is that the distal end side engagement part 51 includes a function of the inner joint part 70. Since other configurations are similar to those of the guide wire 1 of the first embodiment, description thereof is omitted. A distal end side engagement part 51L of the eleventh embodiment is an annular projection part formed in the proximal end part of the distal end coil body 30, and the proximal end part of the distal end coil body 30 is fixed to the core shaft 10. The outer diameter of the distal end side engagement part 51L is larger than the outer diameter of the distal end coil body 30 and the core shaft 10, and engages with the distal end side engaged part 61 to regulate movement of the core shaft 10 of the coil body 20 in the proximal end direction. Even in this case, the relative positions of the coil body 20 and the core shaft can be kept in an appropriate range, and the procedure using the guide wire can be facilitated. By relatively moving the coil body 20 with respect to the core shaft 10, the rigidity of the distal end side of the guide wire 1 can be changed.

### <Twelfth Embodiment>

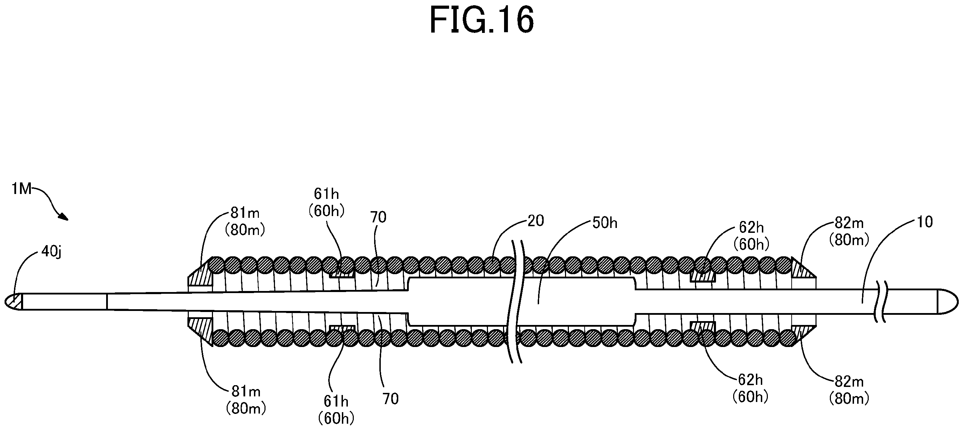

Fig. 16 is an explanatory diagram illustrating an overall configuration of a guide wire 1M of a twelfth embodiment. The configuration of the guide wires illustrated from the first embodiment to the eleventh embodiment may be combined as appropriate. For example, as the guide wire 1M of the twelfth embodiment shown in Fig. 16, the configuration including the engaged part 50h of the eighth embodiment and the configuration not including the distal end coil body 30 as in the ninth embodiment may be combined. Even in this case, by relatively moving the coil body 20 with respect to the core shaft 10, the rigidity of the distal end side of the guide wire 1 can be changed. As another example, the distal end side engaged part 61 may be provided in the distal end of the coil body 20 as the first embodiment, and the proximal end side engaged part 62g may be provided in the inside of the coil body 20 as the eleventh embodiment.

### <Thirteenth Embodiment>

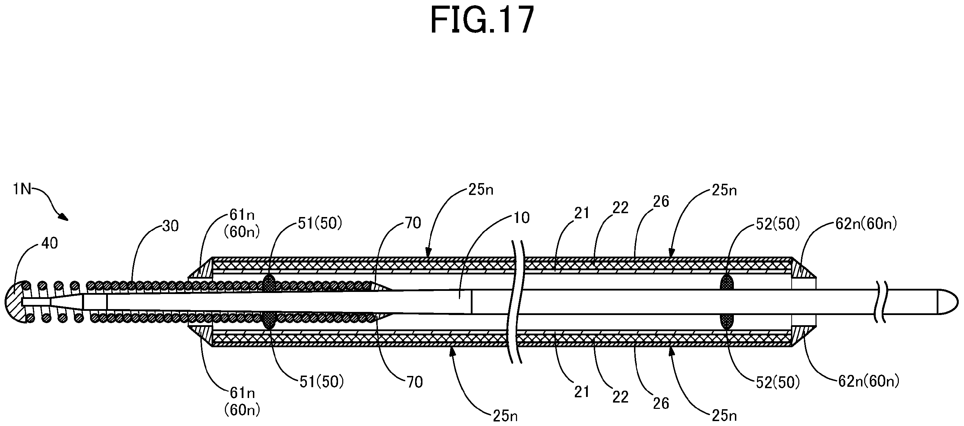

Fig. 17 is an explanatory diagram illustrating an overall configuration of a guide wire IN of a thirteenth embodiment. A difference of the guide wire IN of thirteenth embodiment from the guide wire 1 (Fig. 1) of the first embodiment is that a cylindrical body 25n is provided instead of the coil body 20. Since other configurations are similar to those of the guide wire 1 of the first embodiment, description thereof is omitted. The cylindrical body 25n of the thirteenth embodiment includes an inner layer 21, a reinforcing layer 22, and an outer layer 26.

As similar to the coil body 20 of the first embodiment, the cylindrical body 25n is not fixed to the core shaft 10, and is configured to be relatively movable (slidable) with respect to the core shaft 10. That is, the cylindrical body 25n is configured to be able to reciprocate along the axis line direction of the core shaft 10. Furthermore, the cylindrical body 25n is configured to be relatively rotatable (coaxially rotatable) with respect to the core shaft 10. That is, the cylindrical body 25n is configured to be rotatable with the axis line of the core shaft 10 as the rotation axis.

The inner layer 21 is a tube formed of a resin, and has the core shaft 10 arranged in the inside. The resin material forming the inner layer 21 is not limited particularly, but can be, for example, polytetrafluoroethylene (PTFE), polyvinylidene difluoride (PVDF), perfluoro alkoxyl alkane (PFA), fluorinated ethylene propylene (FEP), ethylene tetrafluoro ethylene (ETFE), polyethylene (PE), or polypropylene (PP). In the present embodiment, the inner layer 21 is formed of the PTFE. For example, when the guide wire IN is inserted to the blood vessel for use, there is a case where a blood clot or the like flows into the inside of the cylindrical body 25n from the distal end side of the cylindrical body 25n. Even in this case, if the inner layer 21 is formed of the PTFE, a blood clot can be particularly prevented from adhering to the inner layer 21, and the smoothness of the relative movement and the relative rotation of the cylindrical body 25n due to adhesion can be prevented from decreasing.

The reinforcing layer 22 is a braided body (metal braid layer) in which a first strand and a second strand are braided with each other in a net shape (mesh shape), arranged in the outer circumference of the inner layer 21, and is covered by (embedded in) the outer layer 26. The cylindrical body 25n includes the reinforcing layer 22 having high rigidity, so that the thickness of the cylindrical body 25n can be made thin while securing the bending rigidity of the cylindrical body 25n, and the outer diameter of the cylindrical body 25n can be made small. The first strand and the second strand may be a round wire or a flat wire. Furthermore, one of the first strand and the second strand may be a round wire, and the other may be a flat wire. Note that, although the reinforcing layer 22 of the present embodiment covers the entire inner layer 21, the reinforcing layer 22 may be configured to cover a part of the inner layer 21. Furthermore, although the reinforcing layer 22 of the present embodiment has constant bending rigidity from the distal end side to the proximal end side, the bending rigidity of the reinforcing layer 22 may be changed in the distal end side and the proximal end side by changing the pitch or the outer diameter of the first strand and the second strand, for example. The braid structure of the reinforcing layer 22 may not be braiding and may be coiling. Furthermore, for example, combination of coiling for the distal end side and braiding for the proximal end side may be adopted.

The outer layer 26 is formed of a resin, and covers the inner layer 21 and the reinforcing layer 22. The resin material forming the outer layer 26 is not limited particularly, but can be polyamide, polyamide elastomer, polyester, polyurethane, polyurethane elastomer or the like, for example. The resin outer layer 26 is formed in the outside of the reinforcing layer 22, so that, for example, when the guide wire IN is inserted to the blood vessel and advances, the friction force generated due to contact of the outer circumference of the guide wire IN and the blood vessel inner wall can be reduced. That is, the outer layer 26 is formed in the outside of the reinforcing layer 22, so that the slidability of the guide wire IN can be improved. Note that, although the outer layer 26 of the present embodiment has constant bending rigidity from the distal end side to the proximal end side, the bending rigidity may be changed in the distal end side and the proximal end side by changing the hardness of the resin forming the outer layer 26.

An engaged part 60n has a similar configuration to that of the engaged part 60 of the first embodiment, and includes a distal end side engaged part 61n and a proximal end side engaged part 62n. The distal end side engaged part 61n and the proximal end side engaged part 62n are annular-shape (ring-shape) member jointed to both ends of the cylindrical body 25n, and an end portion of the reinforcing layer 22 enters to the inside of the distal end side engaged part 61n and the proximal end side engaged part 62n. The inner diameter of each of the distal end side engaged part 61n and the proximal end side engaged part 62n is configured to be smaller than the inner diameter of the cylindrical body 25n. As a result, the inner circumferential surfaces of the distal end side engaged part 61n and the proximal end side engaged part 62n project to the inside from the inner circumferential surface of the cylindrical body 25n. When the cylindrical body 25n relatively moves in the proximal end direction (right direction in Fig. 17) with respect to the core shaft 10, the distal end side engaged part 61n engages (contacts) with the distal end side engagement part 51. When the cylindrical body 25n relatively moves in the distal end direction (left direction in Fig. 17) with respect to the core shaft 10, the proximal end side engaged part 62n engages (contacts) with the proximal end side engagement part 52. As similar to the engaged part 60 of the first embodiment, a taper is formed in the outside of each of the distal end side engaged part 61n and the proximal end side engaged part 62n.

As the guide wire IN of the present embodiment described above, the guide wire may include, instead of the coil body 20 (Fig. 1), the cylindrical body 25n including the inner layer 21, the reinforcing layer 22, and the outer layer 26. Even in this case, by relatively moving this cylindrical body 25n with respect to the core shaft 10, the rigidity of the distal end side of the guide wire IN can be changed. Furthermore, according to the guide wire IN of the present embodiment, since the inner layer 21 is arranged in the inside of the cylindrical body 25n, when the guide wire IN is used in the lumen of a human, a blood clot is prevented from adhering to the inside of the cylindrical body 25n. As a result, the smoothness of the relative movement and the relative rotation of the cylindrical body 25n due to adhesion of a blood clot can be prevented from decreasing. Furthermore, according to the guide wire IN of the present embodiment, since the outer layer 26 is arranged in the outside of the cylindrical body 25n, when the guide wire IN is used in the lumen of a human, the friction force generated due to the contact of the outer circumference of the cylindrical body 25n and the lumen inner wall of a human can be reduced. Furthermore, according to the guide wire IN of the present embodiment, since the cylindrical body 25n includes the reinforcing layer 22 having high rigidity, the thickness of the cylindrical body 25n can be reduced while securing the bending rigidity of the cylindrical body 25n. As a result, the outer diameter of the cylindrical body 25n can be made small.

### <Fourteenth Embodiment>

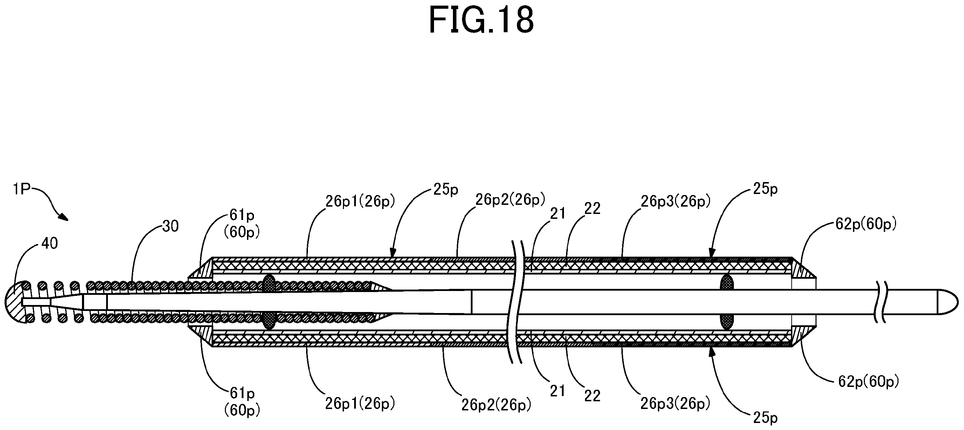

Fig. 18 is an explanatory diagram illustrating an overall configuration of a guide wire 1P of a fourteenth embodiment. A difference of the guide wire 1P of the fourteenth embodiment from the guide wire IN (Fig. 13) of the thirteenth embodiment is that the rigidity changes in the distal end side and the proximal end side of the outer layer 26. Since other configurations are similar to those of the guide wire IN of the thirteenth embodiment, description thereof is omitted. The cylindrical body 25p of the fourteenth embodiment includes the inner layer 21, the reinforcing layer 22, and the outer layer 26p. Since the inner layer 21 and the reinforcing layer 22 are similar to those of the thirteenth embodiment, description thereof is omitted.

The outer layer 26p is formed of a resin, and covers the inner layer 21 and the reinforcing layer 22. The resin material forming the outer layer 26p is not limited particularly, but the same resin as the resin exemplified in the thirteenth embodiment can be adopted. The outer layer 26p has a configuration of a plurality of continuing sections having resin hardness different from each other. Here, in the outer layer 26p, the outer layer of the most distal end side first section is referred to as a "first outer layer 26p1", the outer layer of a second section adjacent to the first outer layer 26p1 in the proximal end side is referred to as a "second outer layer 26p2", and the outer layer of the most proximal end side third section is referred to as a "third outer layer 26p3". That is, the outer layer 26p has a configuration in which the first outer layer 26p1, the second outer layer 26p2, and the third outer layer 26p3 are arrayed from the distal end side to the proximal end side. Note that, here, although the outer layer 26p includes three types of outer layers having resin hardness different from each other, the number of sections having different resin hardness of the outer layer 26p is not limited to three, and may be two, or four or more.

The outer layer 26p is configured so that the resin hardness is reduced from the proximal end side to the distal end side. That is, when the resin hardness of the first outer layer 26p1 is H1, the resin hardness of the second outer layer 26p2 is H2, and the resin hardness of the third outer layer 26p3 is H3, H1 < H2 < H3 is satisfied. Note that, in the present embodiment, the "resin harness" is not limited to hardness of a resin itself, and means the overall hardness obtained by adding the hardness of a material (for example, tungsten powder) mixed to the resin to the hardness of the resin itself.

According to the guide wire 1P of the present embodiment described above, since the cylindrical body 25p is configured so that the hardness of the outer layer 26p is reduced from the proximal end side to the distal end side, the bending rigidity is reduced from the proximal end side to the distal end side. By the distal end side having relatively high flexibility, the guide wire 1P can be hard to damage the blood vessel inner surface even in a branched portion of a steep angle in a blood vessel. On the other hand, by the proximal end side having relatively high rigidity, the torque transmission property of transmitting the rotation operation of the guide wire by a technician to the distal end part side can be increased.

### <Fifteenth Embodiment>

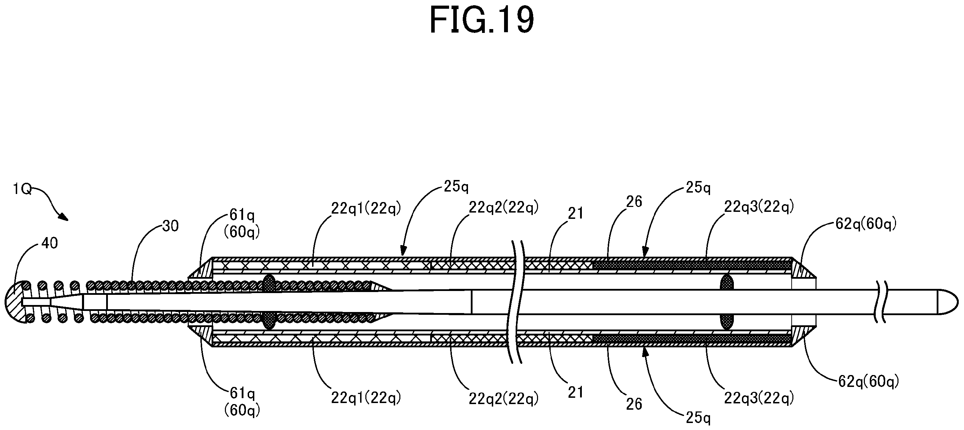

Fig. 19 is an explanatory diagram illustrating an overall configuration of a guide wire 1Q of a fifteenth embodiment. A difference of the guide wire 1Q of the fifteenth embodiment from the guide wire IN (Fig. 13) of the thirteenth embodiment is that the rigidity changes in the distal end side and the proximal end side of the reinforcing layer 22. Since other configurations are similar to those of the guide wire IN of the thirteenth embodiment, description thereof is omitted. The cylindrical body 25q of the fifteenth embodiment includes the inner layer 21, a reinforcing layer 22q, and the outer layer 26. Since the inner layer 21 and the outer layer 26 are similar to those of the thirteenth embodiment, description thereof is omitted.

The reinforcing layer 22q is a braided body (metal braid layer) in which the first strand and the second strand are braided with each other in a net shape (mesh shape), arranged in the outer circumference of the inner layer 21, and is covered by (embedded in) the outer layer 26. The reinforcing layer 22q has a configuration of a plurality of continuing sections having bending rigidity different from each other. Here, in the reinforcing layer 22q, the reinforcing layer of the most distal end side first section is referred to as a "first reinforcing layer 22q1", the reinforcing layer of a second section adjacent to the first reinforcing layer 22q1 in the proximal end side is referred to as a "second reinforcing layer 22q2", and the reinforcing layer of the most proximal end side third section is referred to as a "third reinforcing layer 22q3". That is, the reinforcing layer 22q has a configuration in which the first reinforcing layer 22q1, the second reinforcing layer 22q2, and the third reinforcing layer 22q3 are arrayed from the distal end side to the proximal end side.

The reinforcing layer 22q is configured so that the bending rigidity is reduced from the proximal end side to the distal end side. That is, when the bending rigidity of the first reinforcing layer 22q1 is R1, the bending rigidity of the second reinforcing layer 22q2 is R2, and the bending rigidity of the third reinforcing layer 22q3 is R3, R1 < R2 < R3 is satisfied. The bending rigidity of the first reinforcing layer 22q1, the second reinforcing layer 22q2, and the third reinforcing layer 22q3 can be changed by changing the pitch or the strand diameter of the first strand and the second strand, for example. Note that, here, although the reinforcing layer 22q includes three types of reinforcing layers having bending rigidity different from each other, the number of sections having different bending rigidity of the reinforcing layer 22q is not limited to three, and may be two, or four or more.

According to the guide wire 1Q of the present embodiment described above, the cylindrical body 25p is configured so that the bending rigidity of the reinforcing layer 22q is reduced from the proximal end side to the distal end side. By the distal end side having relatively high flexibility, the guide wire 1Q can be hard to damage the blood vessel inner surface even in a branched portion of a steep angle in a blood vessel. On the other hand, by the proximal end side having relatively high rigidity, the torque transmission property of transmitting the rotation operation of the guide wire by a technician to the distal end part side can be increased.

### <Sixteenth Embodiment>

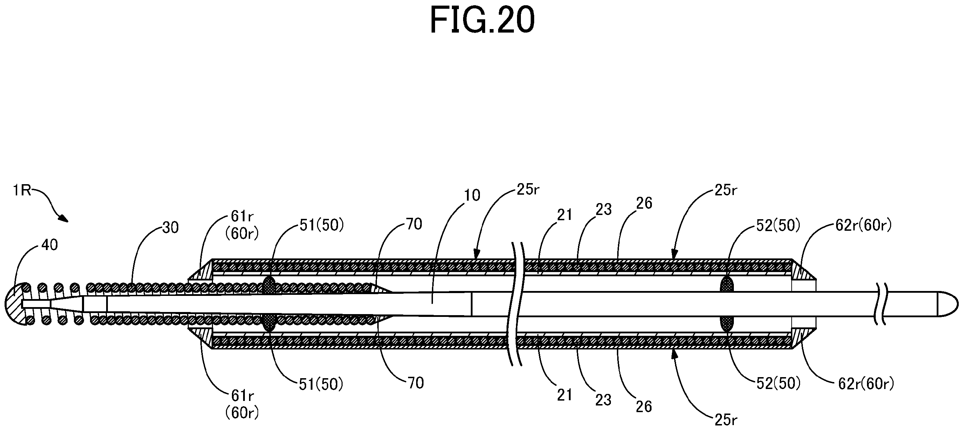

Fig. 20 is an explanatory diagram illustrating an overall configuration of a guide wire 1R of a sixteenth embodiment. A difference of the guide wire 1R of the sixteenth embodiment from the guide wire IN (Fig. 13) of the thirteenth embodiment is that the coil body is provided instead of the reinforcing layer. Since other configurations are similar to those of the guide wire IN of the thirteenth embodiment, description thereof is omitted. A cylindrical body 25r of the sixteenth embodiment includes the inner layer 21, the coil body 23, and the outer layer 26. Since the inner layer 21 and the outer layer 26 are similar to those of the thirteenth embodiment, description thereof is omitted.

The coil body 23 includes one or a plurality of coils, and is covered by (embedded in) the outer layer 26 in a state of being wound around the outer circumference of the inner layer 21. The coil included in the coil body 23 may be a single coil obtained by forming a cylindrical shape by spirally winding one strand having a circular cross section, or may be a hollow twisted wire coil obtained by forming a cylindrical shape by a twisted wire formed of a plurality of strands twisted. The coil body 23 may be formed by combining a single coil and a hollow twisted wire coil. The coil body 23 may be formed of a radiotranslucent alloy such as a stainless alloy (SUS302, SUS304, SUS316 or the like), a super-elastic alloy such as an Ni-Ti alloy, a piano wire, a nickel-chromium alloy, or a cobalt alloy, or a radiopaque alloy such as a gold, a platinum, a tungsten, or an alloy containing these elements (for example, a platinum-nickel alloy). The coil body 23 may be formed of a known material other than those described above. Although the coil body 23 of the present embodiment has a constant coil pitch from the distal end side to the proximal end side, the coil pitch may change.

An engaged part 60r has a similar configuration to that of the engaged part 60 of the first embodiment, and includes a distal end side engaged part 61r and a proximal end side engaged part 62r. The distal end side engaged part 61r and the proximal end side engaged part 62r are annular-shape (ring-shape) member jointed to both ends of the cylindrical body 25r, and an end portion of the coil body 23 enters to the inside of the distal end side engaged part 61r and the proximal end side engaged part 62r. Since other configurations of the distal end side engaged part 61r and the proximal end side engaged part 62r are similar to those of the engaged part 60 of the first embodiment, description thereof is omitted.

As the guide wire 1R of the present embodiment described above, the guide wire may include, instead of the coil body 20 (Fig. 1), the cylindrical body 25r including the inner layer 21, the coil body 23, and the outer layer 26. Even in this case, by relatively moving this cylindrical body 25r with respect to the core shaft 10, the rigidity of the distal end side of the guide wire 1R can be changed. Furthermore, according to the guide wire 1R of the present embodiment, since the inner layer 21 is arranged in the inside of the cylindrical body 25r, as similar to the guide wire IN (Fig. 13) of the thirteenth embodiment, a blood clot is prevented from adhering to the inside of the cylindrical body 25r. Furthermore, according to the guide wire 1R of the present embodiment, since the outer layer 26 is arranged in the outside of the cylindrical body 25r, the friction force generated due to the contact of the outer circumference of the cylindrical body 25r and the lumen inner wall of a human can be reduced. Furthermore, according to the guide wire 1R of the present embodiment, since the cylindrical body 25r includes the coil body 23 having high rigidity, the thickness of the cylindrical body 25r can be reduced while securing the bending rigidity of the cylindrical body 25r. As a result, the outer diameter of the cylindrical body 25r can be made small.

### <Seventeenth Embodiment>

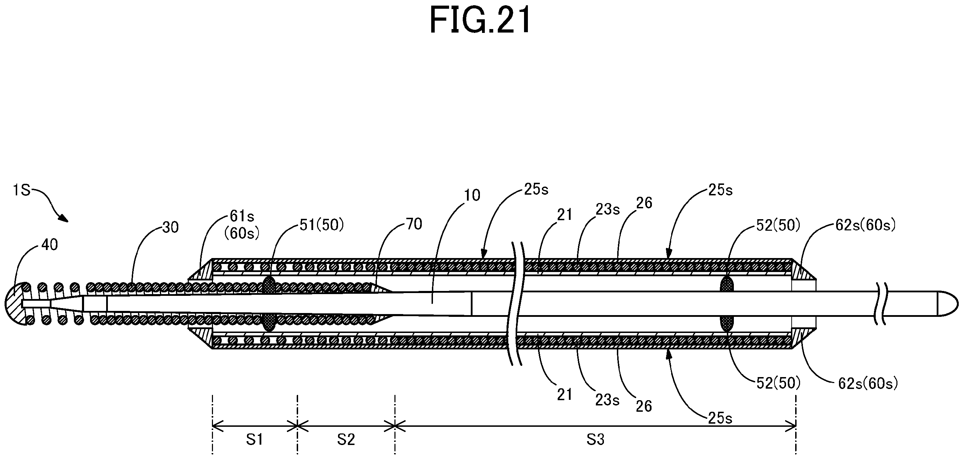

Fig. 21 is an explanatory diagram illustrating an overall configuration of a guide wire IS of a seventeenth embodiment. A difference of the guide wire IS of the seventeenth embodiment from the guide wire 1R (Fig. 20) of the sixteenth embodiment is that the rigidity changes in the distal end side and the proximal end side of the coil body 23. Since other configurations are similar to those of the guide wire 1R of the sixteenth embodiment, description thereof is omitted. The cylindrical body 25s of the seventeenth embodiment includes the inner layer 21, a coil body 23s, and the outer layer 26. Since the inner layer 21 and the outer layer 26 are similar to those of the sixteenth embodiment, description thereof is omitted.

As similar to the coil body 23 of the sixteenth embodiment, the coil body 23s includes one or a plurality of coils, and is covered by (embedded in) the outer layer 26 in a state of being wound around the outer circumference of the inner layer 21. The cross-sectional shape, configuration, and material of the coil body 23s are similar to those of the coil body 23 of the sixteenth embodiment, description thereof is omitted. The coil body 23s has a configuration of a plurality of continuing sections having a coil pitch different from each other. Here, in the coil body 23s, the section of the most distal end side is referred to as a "first section S1", the section adjacent to the first section S1 in the proximal end side is referred to as a "second section S2", and the section of the most proximal end side is referred to as a "third section S3". That is, the coil body 23s has a configuration in which the first section S1, the second section S2, and the third section S3 are arrayed from the distal end side to the proximal end side.

The coil body 23s is configured so that the coil pitch increases from the proximal end side to the distal end side. That is, when the coil pitch of the first section S1 is P1, the coil pitch of the second section S2 is P2, and the coil pitch of the third section S3 is P3, P1 > P2 > P3 is satisfied. Since the coil body 23s is sparsely wound in the distal end side as compared to the proximal end side, the distal end side can be easy to bend. By the distal end side having relatively high flexibility, the coil body 23s can be hard to damage the blood vessel inner surface even in a branched portion of a steep angle in a blood vessel. On the other hand, since the proximal end side is relatively sparsely wound, the torque transmission property of transmitting the rotation operation of the guide wire by a technician to the distal end part side can be increased. Note that, here, although the coil body 23s includes three sections having a coil pitch different from each other, the number of sections having different coil pitches of the coil body 23s is not limited to three, and may be two, or four or more. Furthermore, in the coil body 23s, the coil pitch may change continuously.

According to the guide wire IS of the present embodiment described above, in the coil body 23s of the cylindrical body 25s, the coil pitch may change in the distal end side and the proximal end side. By increasing (widening) the coil pitch, the flexibility of the cylindrical body 25s can be increased. On the other hand, by decreasing (narrowing) the coil pitch, the torque transmission property of the cylindrical body 25s can be increased.

### <Eighteenth Embodiment>

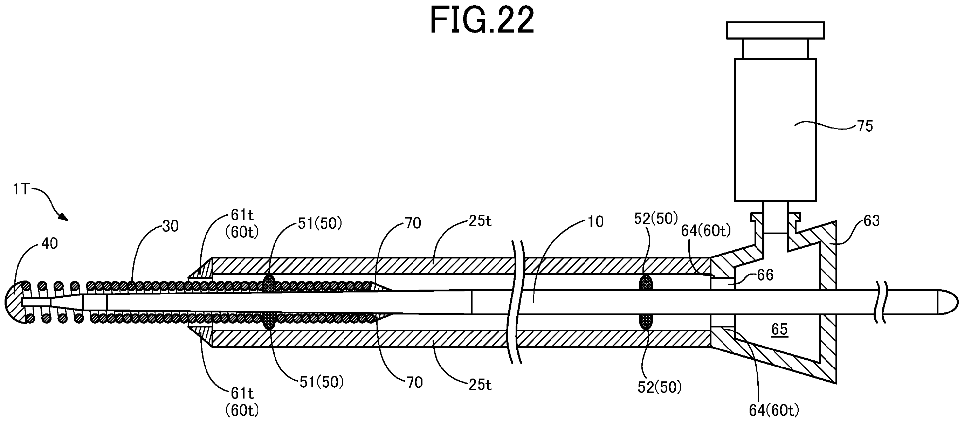

Fig. 22 is an explanatory diagram illustrating an overall configuration of a guide wire 1T of an eighteenth embodiment. Main differences of the guide wire 1T of the eighteenth embodiment from the guide wire 1 (Fig. 1) of the first embodiment are that a cylindrical body 25t is provided instead of the coil body 20, and that a connector 63 is provided instead of the proximal end side engaged part 62. Since other configurations are similar to those of the guide wire 1 of the first embodiment, description thereof is omitted. The guide wire 1T of the eighteenth embodiment includes the cylindrical body 25t, the connector 63, and a syringe 75.

The cylindrical body 25t is a tube formed of a resin, and has the core shaft 10 arranged inside thereof. The resin material forming the cylindrical body 25t is not limited particularly, but can be, for example, polytetrafluoroethylene (PTFE), polyvinylidene difluoride (PVDF), perfluoro alkoxyl alkane (PFA), fluorinated ethylene propylene (FEP), ethylene tetrafluoro ethylene (ETFE), polyethylene (PE), or polypropylene (PP). As similar to the coil body 20 of the first embodiment, the cylindrical body 25t is not fixed to the core shaft 10, and is configured to be able to reciprocate along the axis line direction of the core shaft 10. The cylindrical body 25 is configured to be relatively rotatable (coaxially rotatable) with respect to the core shaft 10.

The connector is a resin member fixed to the proximal end of the cylindrical body 25t, and is configured to relatively move with respect to the core shaft 10, and relatively rotate (coaxially rotate) with respect to the core shaft 10. The connector 63 has a connector engaged part 64, a space part 65, and an opening part 66. The space part 65 is formed in the inside of the core shaft 10, and communicates with the inside of the cylindrical body 25t via the opening part 66. The proximal end side of the core shaft 10 extends from the inside of the cylindrical body 25t through the opening part 66 and the space part 65 to be exposed from the proximal end side of the connector 63 to the outside. The syringe 75 is connected to the connector 63, and a distal end discharge port of the syringe 75 and the space part 65 of the connector 63 communicate with each other. A fluid jet from the syringe 75 passes from the distal end discharge port of the syringe 75 through the space part 65 and the opening part 66 of the connector 63 to be supplied to the inside of the cylindrical body 25t.

The connector engaged part 64 is a portion formed in the outer circumference of the opening part 66, and is connected to the proximal end of the cylindrical body 25t. The opening diameter of the opening part 66, that is, the inner diameter of the connector engaged part 64 is configured to be smaller than the inner diameter of the cylindrical body 25t. As a result, the inner circumferential surface of the connector engaged part 64 projects to the inside from the inner circumferential surface of the cylindrical body 25t. The inner diameter of the connector engaged part 64 (the opening diameter of the opening part 66) is smaller than the outer diameter of the proximal end side engagement part 52 provided in the core shaft 10. As a result, when the cylindrical body 25t relatively moves in the distal end direction (left direction of Fig. 22) with respect to the core shaft 10, the connector engaged part 64 engages (contacts) with the proximal end side engagement part 52.

The distal end side engaged part 61t is attached to the distal end of the cylindrical body 25t. Since the shape and the function of the distal end side engaged part 61t are similar to those of the distal end side engaged part 61 of the first embodiment, description thereof is omitted. The distal end side engaged part 61t and the connector engaged part 64 of the present embodiment are stoppers that engage with the engagement part 50 of the core shaft 10, and are also referred to as the engaged parts 60t as similar to the engaged part 60 (the distal end side engaged part 61 and the proximal end side engaged part 62) of the first embodiment.

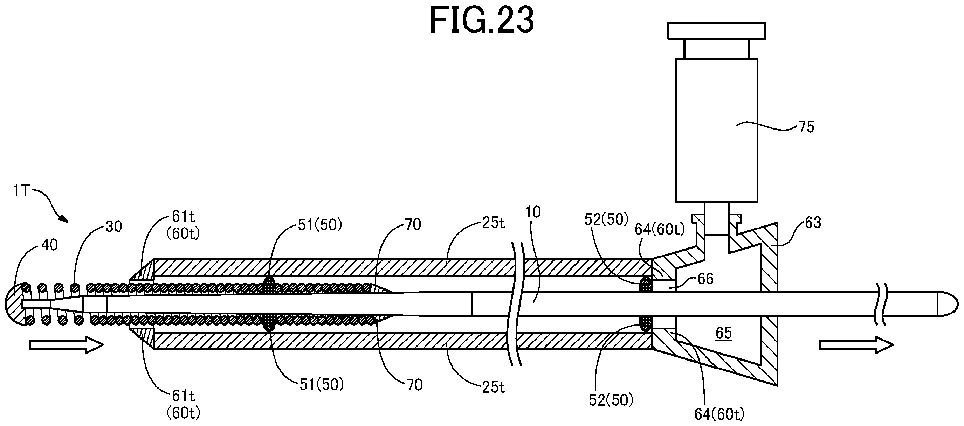

Fig. 23 is an explanatory diagram illustrating a state where the cylindrical body 25t and the connector 63 are relatively moved in the distal end direction (distal end side) of the core shaft 10. The cylindrical body 25t is configured to be able to reciprocate along the axis line direction of the core shaft 10 in a state of covering the distal end side engagement part 51 and the proximal end side engagement part 52. That is, in the guide wire 1T, the length (projection amount) of the small diameter portion including the distal end coil body 30 projecting from the distal end of the cylindrical body 25t and the distal end joint part 40 can be changed without changing the positions of the cylindrical body 25t and the connector 63, and this small diameter portion can be relatively rotated with respect to the cylindrical body 25t.

As shown in Fig. 23, when the cylindrical body 25t and the connector 63 are relatively moved in the distal end direction of the core shaft 10, the connector engaged part 64 contacts the proximal end side engagement part 52 to regulate further movement of the cylindrical body 25t and the connector 63 in the distal end direction. At this time, the length (projection amount) of the small diameter portion including the distal end coil body 30 projecting from the distal end of the cylindrical body 25t and the distal end joint part 40 is the minimum. On the other hand, although not shown in the drawing, when the cylindrical body 25t and the connector 63 are relatively moved in the proximal end direction of the core shaft 10, the distal end engaged part 61t contacts the distal end side engagement part 51 to regulate further movement of the cylindrical body 25t and the connector 63 in the proximal end direction. At this time, the length (projection amount) of the small diameter portion including the distal end coil body 30 projecting from the distal end of the cylindrical body 25t and the distal end joint part 40 is the maximum.

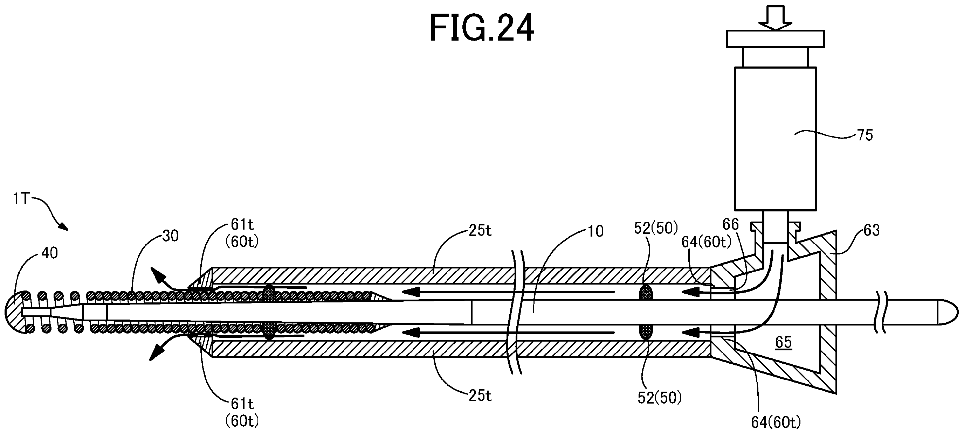

Fig. 24 is a diagram for explaining flushing using a fluid in the syringe. The fluid jet from the syringe 75 by pressing of a piston passes through the space part 65 and the opening part 66 of the connector 63 to be supplied from the proximal end side of the cylindrical body 25t to the inside of the cylindrical body 25t. The fluid supplied to the inside of the cylindrical body 25t flows to the distal end side of the cylindrical body 25t, and is released to the outside of the cylindrical body 25t via the opening part of the distal end side of the cylindrical body 25t. At this time, a foreign matter such as a blood clot existing in the inside of the cylindrical body 25t can be discharged to the outside of the cylindrical body 25 together with the fluid. When the guide wire 1T is inserted to the blood vessel and used, a blood clot or the like flows from the distal end side of the cylindrical body 25t to the inside of the cylindrical body 25t so that the blood clot or the like adheres to the inner layer 21 to reduce the smoothness of the relative movement and the relative rotation of the cylindrical body 25, in some cases. By flowing the fluid jet from the syringe 75 to the inside of the cylindrical body 25t, the inside of the cylindrical body 25t can be flushed so as to prevent smoothness of the relative movement and the relative rotation of the cylindrical body 25t from decreasing.

As the guide wire 1T of the present embodiment described above, a part of the connector 63 may be configured as an engaged part (connector engaged part 64) that engages with the proximal end side engagement part 52. Even in this case, when the cylindrical body 25t is relatively moved in the distal end direction of the core shaft 10, the connector engaged part 64 can contact the proximal end side engagement part 52 to regulate further movement of the cylindrical body 25t in the distal end direction. Furthermore, according to the guide wire 1T of the present embodiment, since the inside of the cylindrical body 25t can be flushed using the fluid in the syringe 75, even when a foreign matter such as a blood clot adheres to the inner layer 21, the foreign matter can be discharged to the outside of the cylindrical body 25t.

### <Nineteenth Embodiment>

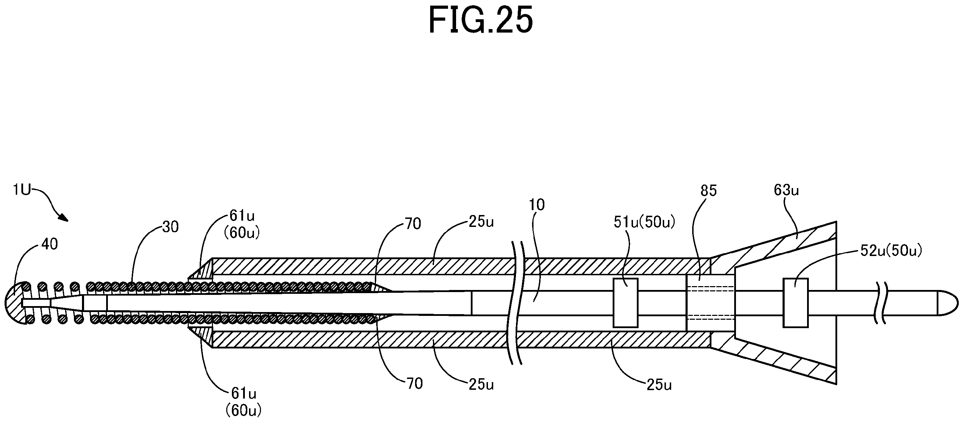

Fig. 25 is an explanatory diagram illustrating an overall configuration of a guide wire 1U of a nineteenth embodiment. Main differences of the guide wire 1U of the nineteenth embodiment from the guide wire 1 (Fig. 1) of the first embodiment are that a cylindrical body 25u is provided instead of the coil body 20, and that the configurations of the engagement part 50 and the engaged part 60 are different. Since other configurations are similar to those of the guide wire 1 of the first embodiment, description thereof is omitted. The guide wire 1U of the nineteenth embodiment includes a cylindrical body 25u, a connector 63u, a distal end side engagement part 51u, a proximal end side engagement part 52u, and an engaged part 85.

Each of the distal end side engagement part 51u and the proximal end side engagement part 52u is a metal member having a cylindrical shape, has the core shaft 10 inserted through the inside, and is fixed to the rear end side from the inner joint part 70 in the core shaft 10 by welding or the like. For the distal end side engagement part 51u and the proximal end side engagement part 52u, the proximal end side engagement part 52u is located in the rear end side from the distal end side engagement part 51u. The outer diameters of the distal end side engagement part 51u and the proximal end side engagement part 52u are larger than the outer diameter of the core shaft 10, and smaller than the inner diameter of the cylindrical body 25u. In other words, the distal end side engagement part 51u and the proximal end side engagement part 52u are annular projection part formed along the outer circumferential direction of the core shaft, and by these, the core shaft 10 has a shape in which the diameter is partially increased. The distal end side engagement part 51u and the proximal end side engagement part 52u of the present embodiment are stoppers that engage with the engaged part 85, and are also referred to as engagement parts 50u as similar to the engagement part 50 (the distal end side engagement part 51 and the proximal end side engagement part 52) of the first embodiment.

The engaged part 85 is a metal member having a cylindrical shape, has the core shaft 10 inserted to the inside, and is arranged between the distal end side engagement part 51u and the proximal end side engagement part 52u in the core shaft 10. The engaged part 85 is not fixed to the core shaft 10, and is configured to be relatively movable (slidable) with respect to the core shaft 10. Furthermore, the engaged part 85 is configured to be relatively rotatable (coaxially rotatable) with respect to the core shaft 10. When the engaged part 85 relatively moves in the proximal end direction of the core shaft 10, the engaged part 85 contacts the proximal end side engagement part 52u to regulate further movement in the proximal end direction.

A connection part between the cylindrical body 25u and the connector 63 is fixed to the outer circumference of the engaged part 85. In other words, the each of the proximal end part of the cylindrical body 25u and the distal end part of the connector 63 is bonded and fixed to the outer circumference of the engaged part 85. Although the cylindrical body 25u and the connector 63 of the present embodiment are directly fixed to each other, the cylindrical body 25u and the connector 63 may not be directly fixed to each other, and may be indirectly fixed to each other.

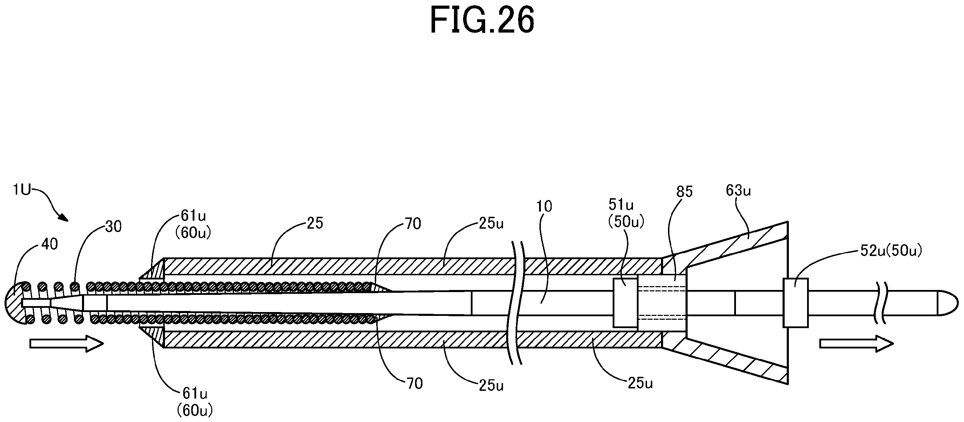

Fig. 26 is an explanatory diagram illustrating a state where the cylindrical body 25u and the connector 63u are relatively moved in the distal end direction (distal end side) of the core shaft 10. The cylindrical body 25u and the connector 63u are configured to be able to reciprocate along the axis line direction of the core shaft 10 in a range in which the engaged part 85 is located between the distal end side engagement part 51u and the proximal end side engagement part 52u. That is, in the guide wire 1U, the length (projection amount) of the small diameter portion including the distal end coil body projecting from the distal end of the cylindrical body 25u and the distal end joint part 40 can be changed without changing the positions of the cylindrical body 25u and the connector 63u, and the small diameter portion can be relatively rotated with respect to the cylindrical body 25u.

As shown in Fig. 26, the cylindrical body 25u and the connector 63u are relatively moved in the distal end direction of the core shaft 10, the engaged part 85 contacts the distal end side engagement part 51u to regulate further movement in the distal end direction of the cylindrical body 25u and the connector 63u. At this time, the length (projection amount) of the small diameter portion including the distal end coil body 30 projecting from the distal end of the cylindrical body 25u and the distal end joint part 40 is the minimum. On the other hand, although not shown in the drawing, when the cylindrical body 25u and the connector 63u are relatively moved in the proximal end direction of the core shaft 10, the engaged part 85 contacts the proximal end side engagement part 52u to regulate further movement in the proximal direction of the cylindrical body 25u and the connector 63u. At this time, the length (projection amount) of the small diameter portion including the distal end coil body 30 projecting from the distal end of the cylindrical body 25u and the distal end joint part 40 is the maximum.

As described above, as the guide wire 1U of the present embodiment, the engaged part that engages with the engagement part 50u provided in the core shaft 10 can be formed by a ring member fixed to the connection part of the cylindrical body 25u and the connector 63u. Even in this case, when the cylindrical body 25u and the core shaft 10 are relatively moved along the axis line direction, the engaged part 85 contacts the distal end side engagement part 51u or the proximal end side engagement part 52u, further movement of the cylindrical body 25u can be regulated.

### <Twelfth Embodiment>

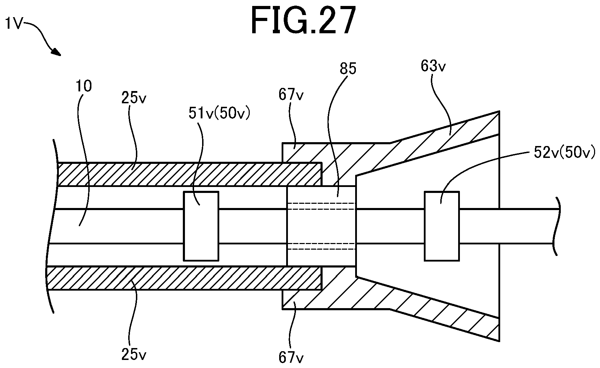

Fig. 27 is an explanatory diagram illustrating the configuration of the proximal end side of a guide wire 1V of a twelfth embodiment. A difference of the guide wire 1V of the twelfth embodiment from the guide wire 1U (Fig. 25) of the nineteenth embodiment is the shape of the connector. Since other configurations are similar to those of the guide wire 1U, description thereof is omitted. In a connector 63v of the twelfth embodiment, the opening diameter of an opening part 67v of the distal end side is larger than the connector 63u of the nineteenth embodiment, and has a proximal end part of a cylindrical body 25v inserted in the inside. As a result, the cylindrical body 25v can be easily fixed to the connector 63v. As similar to the nineteenth embodiment, each of the proximal end part of the cylindrical body 25v and the distal end part of the connector 63v is bonded and fixed to the outer circumference of the engaged part 85. As the guide wire 1V of the present embodiment described above, the connector 63v may be connected to the proximal end part of the cylindrical body 25v so as to cover the proximal end part of the cylindrical body 25v from the outside. Even in this case, the connection part of the cylindrical body 25v and the connector 63v can be fixed to the outer circumference of the engagement part 85.

### <Twenty-First Embodiment>

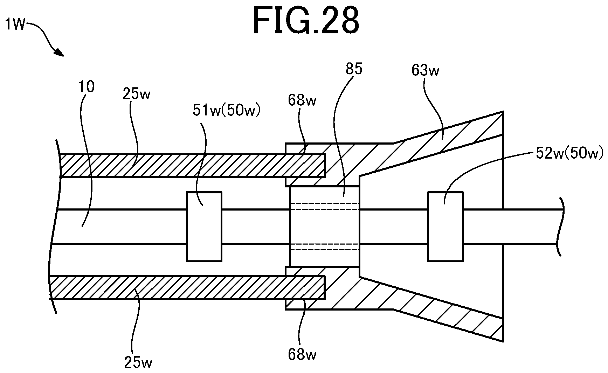

Fig. 28 is an explanatory diagram illustrating a configuration of the proximal end side of a guide wire 1W of a twenty-first embodiment. A difference of the guide wire 1W of the twenty-first embodiment from the guide wire 1U (Fig. 25) of the nineteenth embodiment is the shape of the connector. Since other configurations are similar to those of the guide wire 1U of the nineteenth embodiment, description thereof is omitted. In the connector 63w of the twenty-first embodiment, an annular-shape groove part 68w is formed in the distal end side. The proximal end of a cylindrical body 25w is inserted to this groove part 68w. As a result, the cylindrical body 25w can be easily fixed to the connector 63w. The cylindrical body 25w is not fixed to the outer circumference of the engaged part 85, and the distal end part of the connector 63w is bonded and fixed. As the guide wire 1W of the present embodiment described above, only the connector 63w may be fixed to the engaged part 85, and the cylindrical body 25w may be indirectly fixed to the engaged part 85 via the connector 63w. Even in this case, both the cylindrical body 25w and the connector 63w can be fixed to the engaged part 85.

### <Modification of Present Embodiment>

The present invention is not limited to the embodiments described above, and can be performed in various modes in a range without departing from the gist thereof. For example, the following modifications can be performed.

### [Modification 1]

In the guide wire of the first embodiment, resin coatings may be or may not be formed in the outside of the coil body 20 and the distal end coil body 30. Furthermore, resin coatings of different types may be formed or resin coating of the same type may be formed in the outside of the coil body 20 and the distal end coil body 30.

### [Modification 2]

In the guide wire 1 of the first embodiment, description has been performed as the outer diameters of the distal end side engagement part 51 and the proximal end side engagement part 52 being equal to each other. However, these outer diameters may be different from each other. Furthermore, in the guide wire 1 of the first embodiment, description has been performed as the inner diameters of the distal end side engaged part 61 and the proximal end side engaged part 62 being equal to each other. However, these inner diameters may be different from each other. That is, the distal end side engagement part 51 and the proximal end side engagement part 52 may have the same shape, and may have different shapes. Furthermore, the distal end side engagement part 51 and the proximal end side engagement part 52 may be formed of the same material, or different materials. This is similar for the distal end side engaged part 61 and the proximal end side engaged part 62.

### [Modification 3]

In the guide wire 1 of the first embodiment, description has been performed as the distal end side engagement part 51 and the proximal end side engagement part 52 being annular projection parts. However, these may not have annular shapes. For example, the distal end side engagement part 51 and the proximal end side engagement part 52 may be projection parts projecting from the outer circumferential surface of the core shaft 10 to a normal direction. Furthermore, the distal end side engaged part 61 and the proximal end side engaged part 62 may not have annular shapes. For example, the distal end side engaged part 61 and the proximal end side engaged part 62 may be a projection part formed in a part of the end surface or the inner circumferential surface of the coil body 20 and projecting to the inside.

### [Modification 4]

The coil pitch, inner diameter, and outer diameter of the coil body 20 and the distal end coil body 30 of the first embodiment are not limited particularly, and may have an arbitrary size. The outer diameter of the strand forming the coil body 20 of the present embodiment may be constant or may change. The cross-sectional shape of the strand is not limited to a circle shape, and may be other shape such as a rectangular shape. The number of threads of the coil body 20 may be single or plural. Furthermore, the coil pitch of the coil body 20 may be constant or may change.

### [Modification 5]

The guide wire 1C of the third embodiment may not include the proximal end side engaged part 62c. Alternatively, instead of the proximal end side engaged part 62, the proximal end forming part (for example, the proximal end forming part 82 of the seventh embodiment) having no engagement function may be formed. Furthermore, the guide wire ID of the fourth embodiment may not include the distal end side engaged part 61d. Alternatively, instead of the distal end side engaged part 61d, a distal end forming part (for example, the distal end forming part 81 of the seventh embodiment) having no engagement function may be formed.

### [Modification 6]

The cylindrical body 25 of the fifth embodiment has been described as a tube formed of a resin. However, the cylindrical body 25 may be other than a tube formed of a resin. For example, the cylindrical body 25 may be a metal slit pipe, or a metal mesh member.

### [Modification 7]

The shapes of the distal end side engaged part 61f and the proximal end side engaged part 62f of the sixth embodiment are examples, and may be arbitrary shapes. Furthermore, these may be different shapes from each other.

### [Modification 8]

The configurations of the first to twelfth embodiment described above can be applied to medical equipment other than a guide wire. For example, the configurations of the present embodiments can be applied also to a dilator, endoscope, catheter or the like. Furthermore, parts of configurations of the guide wires exemplified in the first to twelfth embodiments can be combined or removed as appropriate.

### [Modification 9]

The guide wire IN (Fig. 17) of the thirteenth embodiment has been described as including the inner layer 21, the reinforcing layer 22, and the outer layer 26. However, the guide wire IN may not include at least one of the inner layer 21 and the outer layer 26. Furthermore, the guide wire IN may not include the reinforcing layer 22.

### [Modification 10]

The outer layer 26p (Fig. 18) of the fourteenth embodiment and the reinforcing layer 22q (Fig. 19) of the fifteenth embodiment may be combined as appropriate. That is, as a method of changing the bending rigidity of the cylindrical body including the outer layer and the reinforcing layer, the resin hardness of the outer layer may be changed as the outer layer 26p of the fourteenth embodiment, and the bending rigidity of the reinforcing layer may be changed as the reinforcing layer 22q of the fifteenth embodiment. Furthermore, as the outer layer 26 of the cylindrical body 25s (Fig. 21) of the seventeenth embodiment, the outer layer 26p (Fig. 18) of the fourteenth embodiment may be applied.

### [Modification 11]

The cylindrical body 25t (Fig. 22) of the guide wire 1T of the eighteenth embodiment has been described as being formed of a single resin. However, as the cylindrical body 25t of the guide wire 1T, the coil body 20 of the first embodiment or the configuration of the cylindrical body of the thirteenth to seventeenth embodiments may be applied. Furthermore, as the cylindrical body of the guide wire of the nineteenth to twenty-first embodiments, the configuration of the cylindrical body of the thirteenth to seventeenth embodiments may be applied.

### [Modification 12]

The connector 63u (Fig. 25) of the nineteenth embodiment has been described as not including a syringe mounted as the connector 63 (Fig. 22) of the eighteenth embodiment. In this case, the fluid jet from the syringe may pass through the opening part of the inside of the engaged part 85 to be supplied to the inside of the cylindrical body 25u.

### [Modification 13]

The configurations of the thirteenth to twenty-first embodiment described above can be applied also to medical equipment such as a dilator, endoscope, catheter or the like. Furthermore, parts of configurations of the guide wires exemplified in the thirteenth to twenty-first embodiments can be combined or removed as appropriate.

### [Modification 14]

In the guide wires of the first to fourth and sixth to twelfth embodiments, instead of the coil body 20, the configuration of the cylindrical body of the thirteenth to seventeenth embodiments may be applied. Furthermore, in the guide wires of the thirteenth to twenty-first embodiment, as the configuration of the engagement part and the engaged part, the configuration of the engagement part and the engaged part of the first to twelfth embodiment may be applied.

Although the present mode has been described above on the basis of embodiments and modification, the embodiments of the mode described above is for facilitating understanding of the present mode, and not for limitation of the present mode. The present mode can be changed or improved without departing the gist and the scope of the claims, and includes equivalents thereof. Furthermore, unless a technical feature is not described as essential in this specification, the technical feature can be deleted as appropriate.

### Reference Signs List

- 1: Guide wire
- 10: Core shaft
- 20: Coil body
- 25: Cylindrical body
- 30: Distal end coil body
- 40: Distal end joint part
- 50: Engagement part
- 51: Distal end side engagement part
- 52: Proximal end side engagement part
- 60: Engaged part
- 61: Distal end side engaged part
- 62: Proximal end side engaged part
- 70: Inner joint part
- 81: Distal end forming part
- 82: Proximal end forming part
- 91: Guiding catheter
- 92: Support catheter
- 93: Micro catheter

## Claims

1. A guide wire comprising:
a core shaft;
an engagement part provided in outer circumference of the core shaft;
a cylindrical body provided in an outside of the core shaft and movable along an axis line direction of the core shaft; and
an engaged part that is provided in the cylindrical body, and engages with the engagement part when the cylindrical body moves in a first direction along the axis line direction of the core shaft to regulate further movement of the cylindrical body in the first direction.

2. The guide wire in accordance with claim 1,
wherein the engagement part comprises
a first engagement part, and
a second engagement part provided in a proximal end side of the core shaft from the first engagement part, and
the engaged part comprises
a first engaged part that engages with the first engagement part to regulate further movement of the cylindrical body in the first direction when the cylindrical body moves in the first direction, and
a second engaged part that engages with the second engagement part to regulate further movement of the cylindrical body in a second direction when the cylindrical body moves in the second direction along the axis line direction of the core shaft.

3. The guide wire in accordance with claim 2,
wherein a length of the cylindrical body is longer than a distance from the first engagement part to the second engagement part, and
the cylindrical body is movable along the axis line direction of the core shaft in a state of covering the first engagement part and the second engagement part.

4. The guide wire in accordance with claim 2 or 3,
wherein the first engagement part and the second engagement part are annular projection parts formed along an outer circumferential direction of the core shaft.

5. The guide wire in accordance with any one of claims 2 to 4,
wherein the first engaged part is provided in a distal end of the cylindrical body, and
the second engaged part is provided in a proximal end of the cylindrical body.

6. The guide wire in accordance with claim 5,
wherein each of the first engaged part and the second engaged part has an annular outer shape,
a taper having an outer diameter expanding from a distal end side to a proximal end side is formed in the first engaged part, and
a taper having an outer diameter expanding from a proximal end side to a distal end side is formed in the second engaged part.

7. The guide wire in accordance with any one of claims 2 to 6,
further comprising a coil body covering a distal end side of the core shaft,
wherein the cylindrical body covers a part of a proximal end side of the coil body,
the first engagement part is provided between a distal end and a proximal end of the coil body, and
the first engaged part is located in the distal end side of the core shaft from the first engagement part.

8. The guide wire in accordance with any one of claims 1 to 7,
wherein the cylindrical body is a coil body.

9. The guide wire in accordance with any one of claims 1 to 7,
wherein the cylindrical body comprises an inner layer formed of a resin, a reinforcing layer arranged in outer circumference of the inner layer, and an outer layer covering the reinforcing layer.

10. The guide wire in accordance with any one of claims 1 to 7,
wherein the cylindrical body comprises an inner layer formed of a resin, an intermediate coil body arranged in outer circumference of the inner layer, and an outer layer covering the intermediate coil body.
